# EUROPEAN PATENT SPECIFICATION

(11) **EP 4 028 401 B1**
(45) Date of publication and mention of the grant of the patent: **22.11.2023**
(21) Application number: 20772007.9
(22) Date of filing: 07.09.2020
(51) Int. Cl.: C07D 498/04, A61P 25/28, A61P 29/00, A61P 35/00, C07D 487/04, A61K 31/5383

(54) **4,4A,5,7,8,8A-HEXAPYRIDO[4,3-B][1,4]OXAZIN-3-ONE COMPOUNDS AS MAGL INHIBITORS**
4,4A,5,7,8,8A-HEXAPYRIDO[4,3-B][1,4]OXAZIN-3-ON-VERBINDUNGEN ALS MAGL INHIBITOREN
COMPOSÉS DE 4,4A,5,7,8,8A-HEXAPYRIDO[4,3-B][1,4]OXAZIN-3-ONE COMME INHIBITEURS DE MAGL

(30) Priority: 09.09.2019 EP 19196089
(43) Date of publication of application: 20.07.2022
(73) Proprietor: F. Hoffmann-La Roche AG, 4070 Basel (CH)
(72) Inventor: GRETHER, Uwe, 4070 Basel (CH); HORNSPERGER, Benoit, 4070 Basel (CH); KROLL, Carsten, 4070 Basel (CH); KUHN, Bernd, 4070 Basel (CH); LUTZ, Marius Daniel Rinaldo, 4070 Basel (CH); O'HARA, Fionn, 4070 Basel (CH); RICHTER, Hans, 4070 Basel (CH)
(74) Representative: Neuhaus, Christian Michel
(86) International application number: PCT/EP2020/074897
(87) International publication number: WO 2021/048036

(56) References cited:
- WO-A1-2017/087858
- WO-A1-2017/087863
- WO-A1-2019/115660
- WO-A1-2019/134985
- WO-A1-2019/180185
- WO-A1-2020/035424
- WO-A1-2020/035425
- WO-A1-2020/104494

## Description

### Field of the Invention

The present invention relates to organic compounds useful for therapy or prophylaxis in a mammal, and in particular to monoacylglycerol lipase (MAGL) inhibitors for use in the treatment or prophylaxis of neuroinflammation, neurodegenerative diseases, pain, cancer, mental disorders, multiple sclerosis, Alzheimer's disease, Parkinson's disease, amyotrophic lateral sclerosis, traumatic brain injury, neurotoxicity, stroke, epilepsy, anxiety, migraine, depression, inflammatory bowel disease, abdominal pain, abdominal pain associated with irritable bowel syndrome and/or visceral pain in a mammal.

### Background of the Invention

Endocannabinoids (ECs) are signaling lipids that exert their biological actions by interacting with cannabinoid receptors (CBRs), CB1 and CB2. They modulate multiple physiological processes including neuroinflammation, neurodegeneration and tissue regeneration (Iannotti, F.A., et al., Progress in lipid research 2016, 62, 107-28.). In the brain, the main endocannabinoid, 2-arachidonoylglycerol (2-AG), is produced by diacyglycerol lipases (DAGL) and hydrolyzed by the monoacylglycerol lipase, MAGL. MAGL hydrolyses 85% of 2-AG; the remaining 15% being hydrolysed by ABHD6 and ABDH12 (Nomura, D.K., et al., Science 2011, 334, 809.). MAGL is expressed throughout the brain and in most brain cell types, including neurons, astrocytes, oligodendrocytes and microglia cells (Chanda, P.K., et al., Molecular pharmacology 2010, 78, 996; Viader, A., et al., Cell reports 2015, 12, 798.). 2-AG hydrolysis results in the formation of arachidonic acid (AA), the precursor of prostaglandins (PGs) and leukotrienes (LTs). Oxidative metabolism of AA is increased in inflamed tissues. There are two principal enzyme pathways of arachidonic acid oxygenation involved in inflammatory processes, the cyclo-oxygenase which produces PGs and the 5-lipoxygenase which produces LTs. Of the various cyclooxygenase products formed during inflammation, PGE2 is one of the most important. These products have been detected at sites of inflammation, e.g. in the cerebrospinal fluid of patients suffering from neurodegenerative disorders and are believed to contribute to inflammatory response and disease progression. Mice lacking MAGL (Mgll-/-) exhibit dramatically reduced 2-AG hydrolase activity and elevated 2-AG levels in the nervous system while other arachidonoyl-containing phospho- and neutral lipid species including anandamide (AEA), as well as other free fatty acids, are unaltered. Conversely, levels of AA and AA-derived prostaglandins and other eicosanoids, including prostaglandin E2 (PGE2), D2 (PGD2), F2 (PGF2), and thromboxane B2 (TXB2), are strongly decreased. Phospholipase A₂ (PLA₂) enzymes have been viewed as the principal source of AA, but cPLA₂-deficient mice have unaltered AA levels in their brain, reinforcing the key role of MAGL in the brain for AA production and regulation of the brain inflammatory process.

Neuroinflammation is a common pathological change characteristic of diseases of the brain including, but not restricted to, neurodegenerative diseases (e.g. multiple sclerosis, Alzheimer's disease, Parkinson disease, amyotrophic lateral sclerosis, traumatic brain injury, neurotoxicity, stroke, epilepsy and mental disorders such as anxiety and migraine). In the brain, production of eicosanoids and prostaglandins controls the neuroinflammation process. The pro-inflammatory agent lipopolysaccharide (LPS) produces a robust, time-dependent increase in brain eicosanoids that is markedly blunted in Mgll-/- mice. LPS treatment also induces a widespread elevation in pro-inflammatory cytokines including interleukin-1-a (IL-1-a), IL-1b, IL-6, and tumor necrosis factor-a (TNF-a) that is prevented in Mgll-/- mice.

Neuroinflammation is characterized by the activation of the innate immune cells of the central nervous system, the microglia and the astrocytes. It has been reported that anti-inflammatory drugs can suppress in preclinical models the activation of glia cells and the progression of disease including Alzheimer's disease and mutiple sclerosis (Lleo A., Cell Mol Life Sci. 2007, 64, 1403.). Importantly, genetic and/or pharmacological disruption of MAGL activity also blocks LPS-induced activation of microglial cells in the brain (Nomura, D.K., et al., Science 2011, 334, 809.).

In addition, genetic and/or pharmacological disruption of MAGL activity was shown to be protective in several animal models of neurodegeneration including, but not restricted to, Alzheimer's disease, Parkinson's disease and multiple sclerosis. For example, an irreversible MAGL inhibitor has been widely used in preclinical models of neuroinflammation and neurodegeneration (Long, J.Z., et al., Nature chemical biology 2009, 5, 37.). Systemic injection of such inhibitor recapitulates the Mgll-/- mice phenotype in the brain, including an increase in 2-AG levels, a reduction in AA levels and related eicosanoids production, as well as the prevention of cytokines production and microglia activation following LPS-induced neuroinflammation (Nomura, D.K., et al., Science 2011, 334, 809.), altogether confirming that MAGL is a druggable target.

Consecutive to the genetic and/or pharmacological disruption of MAGL activity, the endogenous levels of the MAGL natural substrate in the brain, 2-AG, are increased. 2-AG has been reported to show beneficial effects on pain with, for example, anti-nociceptive effects in mice (Ignatowska-Jankowska B. et al., J. Pharmacol. Exp. Ther. 2015, 353, 424.) and on mental disorders, such as depression in chronic stress models (Zhong P. et al., Neuropsychopharmacology 2014, 39, 1763.).

Furthermore, oligodendrocytes (OLs), the myelinating cells of the central nervous system, and their precursors (OPCs) express the cannabinoid receptor 2 (CB2) on their membrane. 2-AG is the endogenous ligand of CB1 and CB2 receptors. It has been reported that both cannabinoids and pharmacological inhibition of MAGL attenuate OLs's and OPCs's vulnerability to excitotoxic insults and therefore may be neuroprotective (Bernal-Chico, A., et al., Glia 2015, 63, 163.). Additionally, pharmacological inhibition of MAGL increases the number of myelinating OLs in the brain of mice, suggesting that MAGL inhibition may promote differentiation of OPCs in myelinating OLs in vivo (Alpar, A., et al., Nature communications 2014, 5, 4421.). Inhibition of MAGL was also shown to promote remyelination and functional recovery in a mouse model of progressive multiple sclerosis (Feliu A. et al., Journal of Neuroscience 2017, 37 (35), 8385.).

In addition, in recent years, metabolism is talked highly important in cancer research, especially the lipid metabolism. Researchers believe that the de novo fatty acid synthesis plays an important role in tumor development. Many studies illustrated that endocannabinoids have anti-tumorigenic actions, including anti-proliferation, apoptosis induction and anti-metastatic effects. MAGL as an important decomposing enzyme for both lipid metabolism and the endocannabinoids system, additionally as a part of a gene expression signature, contributes to different aspects of tumourigenesis, including in glioblastoma (Qin, H., et al., Cell Biochem. Biophys. 2014, 70, 33; Nomura DK et al., Cell 2009, 140(1), 49-61; Nomura DK et al., Chem. Biol. 2011, 18(7), 846-856, Jinlong Yin et al, Nature Communications 2020, 11, 2978).

The endocannabinoid system is also invlolved in many gastrointestinal physiological and physiopathological actions (Marquez L. et al., PLoS One 2009, 4(9), e6893). All these effects are driven mainly via cannabinoid receptors (CBRs), CB1 and CB2. CB1 receptors are present throughout the GI tract of animals and healthy humans, especially in the enteric nervous system (ENS) and the epithelial lining, as well as smooth muscle cells of blood vessels in the colonic wall (Wright K. et al., Gastroenterology 2005, 129(2), 437-453; Duncan, M. et al., Aliment Pharmacol Ther 2005, 22(8), 667-683). Activation of CB1 produces anti-emetic, anti-motility, and anti-inflammatory effect, and help to modulate pain (Perisetti, A. et al., Ann Gastroenterol 2020, 33(2), 134-144). CB2 receptors are expressed in immune cells such as plasma cells and macrophages, in the lamina propria of the GI tract (Wright K. et al., Gastroenterology 2005, 129(2), 437-453), and primarily on the epithelium of human colonic tissue associated with inflammatory bowel disease (IBD). Activation of CB2 exerts anti-inflammatory effect by reducing pro-inflammatory cytokines. Expression of MAGL is increased in colonic tissue in UC patients (Marquez L. et al., PLoS One 2009, 4(9), e6893) and 2-AG levels are increased in plasma of IBD patients (Grill, M. et al., Sci Rep 2019, 9(1), 2358). Several animal studies have demonstrated the potential of MAGL inhibitors for symptomatic treatment of IBD. MAGL inhibition prevents TNBS-induced mouse colitis and decreases local and circulating inflammatory markers via a CB1/CB2 MoA (Marquez L. et al., PLoS One 2009, 4(9), e6893). Furthermore, MAGL inhibition improves gut wall integrity and intestinal permeability via a CB1 driven MoA (Wang, J. et al., Biochem Biophys Res Commun 2020, 525(4), 962-967). WO2019/115660 discloses 4H-1,4-benzoxazin-3-one compounds as MAGL inhibitors.

In conclusion, suppressing the action and/or the activation of MAGL is a promising new therapeutic strategy for the treatment or prevention of neuroinflammation, neurodegenerative diseases, pain, cancer, mental disorders, inflammatory bowel disease, abdominal pain and abdominal pain associated with irritable bowel syndrome. Furthermore, suppressing the action and/or the activation of MAGL is a promising new therapeutic strategy for providing neuroprotection and myelin regeneration. Accordingly, there is a high unmet medical need for new MAGL inhibitors.

### Summary of the Invention

In a first aspect, the present invention provides a compound of formula (I) 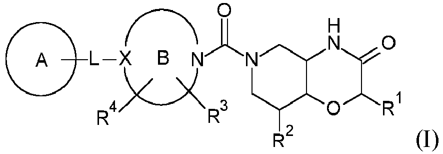 or a pharmaceutically acceptable salt thereof, wherein A, B, L, X, and R¹ to R⁴ are as described herein. Also disclosed herein but not part of the invention is a process of manufacturing the urea compounds of formula (I) described herein, and pharmaceutically acceptable salts thereof, comprising:
(a) reacting a first amine of formula **1**, wherein R¹ and R² are as described herein, preferably wherein R¹ and R² are hydrogen, 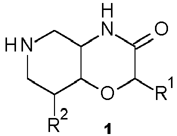 with a secondary amine **2**, wherein A, B, L, X, R³ and R⁴ are as described herein 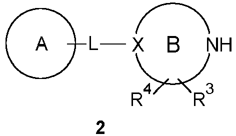 in the presence of a base and a urea forming reagent, to form said compound of formula (I); and optionally
(b) transforming said compound of formula (I) to a pharmaceutically acceptable salts thereof.

In a further aspect, the present invention provides a compound of formula (I) as described herein, for use as therapeutically active substance.

In a further aspect, the present invention provides a pharmaceutical composition comprising a compound of formula (I) as described herein and a therapeutically inert carrier.

In a further aspect, the present invention provides a compound of formula (I) as described herein or of a pharmaceutical composition described herein for use in inhibiting monoacylglycerol lipase (MAGL) in a mammal.

In a further aspect, the present invention provides a compound of formula (I) as described herein or of a pharmaceutical composition described herein for use in the treatment or prophylaxis of neuroinflammation, neurodegenerative diseases, pain, cancer, mental disorders and/or inflammatory bowel disease in a mammal.

In a further aspect, the present invention provides a compound of formula (I) as described herein or of a pharmaceutical composition described herein for use in the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain, spasticity associated with pain, abdominal pain, abdominal pain associated with irritable bowel syndrome and/or visceral pain in a mammal.

### Detailed Description of the Invention

### Definitions

Features, integers, characteristics, compounds, chemical moieties or groups described in conjunction with a particular aspect, embodiment or example of the invention are to be understood to be applicable to any other aspect, embodiment or example described herein, unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The description may encompass subject-matter extending beyond the scope of the claims. However the scope of the invention and of protection is solely defined by the appended claims and shall not extend beyond their scope.

The term "alkyl" refers to a mono- or multivalent, e.g., a mono- or bivalent, linear or branched saturated hydrocarbon group of 1 to 6 carbon atoms ("C₁-C₆-alkyl"), e.g., 1, 2, 3, 4, 5, or 6 carbon atoms. In some embodiments, the alkyl group contains 1 to 3 carbon atoms, e.g., 1, 2 or 3 carbon atoms. Some non-limiting examples of alkyl include methyl, ethyl, propyl, 2-propyl (isopropyl), n-butyl, iso-butyl, sec-butyl, tert-butyl, and 2,2-dimethylpropyl. A particularly preferred, yet non-limiting example of alkyl is methyl.

The term "alkoxy" refers to an alkyl group, as previously defined, attached to the parent molecular moiety via an oxygen atom. Unless otherwise specified, the alkoxy group contains 1 to 6 carbon atoms ("C₁-C₆-alkoxy"). In some preferred embodiments, the alkoxy group contains contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy. A particularly preferred, yet non-limiting example of alkoxy is methoxy.

The term "halogen" or "halo" refers to fluoro (F), chloro (Cl), bromo (Br), or iodo (I). Preferably, the term "halogen" or "halo" refers to fluoro (F), chloro (Cl) or bromo (Br). Particularly preferred, yet non-limiting examples of "halogen" or "halo" are fluoro (F) and chloro (Cl).

The term "bridged bicyclic heterocycle" refers to a chemical entity consisting of two heterocyclyl moieties as defined herein, or to a combination of one heterocyclyl and one cycloalkyl moiety, having two ring atoms in common, i.e., the bridge separating the two rings is either a single bond or a chain of one or two ring atoms. In some embodiments, the bridged bicyclic heterocycle is a 6-14 membered bridged bicyclic heterocycle comprising 1-3 heteroatoms selected from N, S, and O. In some preferred embodiments, the bridged bicyclic heterocycle is a 6-9 membered bridged bicyclic heterocycle comprising 1-2 heteroatoms selected from N and O. In some particularly preferred embodiments, the bridged bicyclic heterocycle is a 6-8 membered bridged bicyclic heterocycle comprising 1 nitrogen atom. Some preferred, yet non-limiting examples of bridged bicyclic heterocycles include 8-azabicyclo[3.2.1]octane-yl; 3,3a,4,5,6,6a-hexahydro-1H-cyclopenta[c]pyrrolyl; 3-azabicyclo[3.1.0]hexanyl; 2-azabicyclo[2.2.1]heptane-yl; 3-azabicyclo[3.2.1]octane-yl; 2,3,3a,4,6,6a-hexahydro-1H-pyrrolo[3,4-c]pyrrolyl; 3-oxa-9-azabicyclo[3.3.1]nonane-yl; and 3-azabicyclo[3.1.1]heptane-yl.

The term "heterocyclyl" refers to a saturated or partly unsaturated monocyclic ring system of 3 to 10 ring atoms, preferably 3 to 8 ring atoms, wherein 1, 2, or 3 of said ring atoms are heteroatoms selected from N, O and S, the remaining ring atoms being carbon. Preferably, 1 to 2 of said ring atoms are selected from N and O, the remaining ring atoms being carbon. Some non-limiting examples of heterocyclyl groups include azetidin-3-yl, azetidin-2-yl, oxetan-3-yl, oxetan-2-yl, 2-oxopyrrolidin-1-yl, 2-oxopyrrolidin-3-yl, 5-oxopyrrolidin-2-yl, 5-oxopyrrolidin-3-yl, 2-oxo-1-piperidyl, 2-oxo-3-piperidyl, 2-oxo-4-piperidyl, 6-oxo-2-piperidyl, 6-oxo-3-piperidyl, 1-piperidinyl, 2-piperidinyl, 3-piperidinyl, 4-piperidinyl, morpholino, morpholin-2-yl and morpholin-3-yl.

The term "cycloalkyl" as used herein refers to a saturated or partly unsaturated monocyclic hydrocarbon group of 3 to 10 ring carbon atoms ("C₃-C₁₀-cycloalkyl"). In some preferred embodiments, the cycloalkyl group is a saturated monocyclic hydrocarbon group of 3 to 8 ring carbon atoms. Preferably, the cycloalkyl group is a saturated monocyclic hydrocarbon group of 3 to 6 ring carbon atoms, e.g., of 3, 4, 5 or 6 carbon atoms. Some non-limiting examples of cycloalkyl include cyclopropyl, cyclobutyl, cyclopentyl, cyclohexyl and cycloheptyl. A particularly preferred, yet non-limiting example of cycloalkyl is cyclopropyl.

The term "aryl" refers to a monocyclic, bicyclic, or tricyclic carbocyclic ring system having a total of 6 to 14 ring members ("C₆-C₁₄-aryl"), preferably, 6 to 12 ring members, and more preferably 6 to 10 ring members, and wherein at least one ring in the system is aromatic. A particularly preferred, yet non-limiting example of aryl is phenyl.

The term "heteroaryl" refers to a mono- or multivalent, monocyclic or bicyclic, preferably bicyclic ring system having a total of 5 to 14 ring members, preferably, 5 to 12 ring members, and more preferably 5 to 10 ring members, wherein at least one ring in the system is aromatic, and at least one ring in the system contains one or more heteroatoms. Preferably, "heteroaryl" refers to a 5-10 membered heteroaryl comprising 1, 2, 3 or 4 heteroatoms independently selected from O, S and N. Most preferably, "heteroaryl" refers to a 5-10 membered heteroaryl comprising 1 to 2 heteroatoms independently selected from O and N. Some non-limiting examples of heteroaryl include 2-pyridyl, 3-pyridyl, 4-pyridyl, indol-1-yl, 1H-indol-2-yl, 1H-indol-3-yl, 1H-indol-4-yl, 1H-indol-5-yl, 1H-indol-6-yl, 1H-indol-7-yl, 1,2-benzoxazol-3-yl, 1,2-benzoxazol-4-yl, 1,2-benzoxazol-5-yl, 1,2-benzoxazol-6-yl, 1,2-benzoxazol-7-yl, 1H-indazol-3-yl, 1H-indazol-4-yl, 1H-indazol-5-yl, 1H-indazol-6-yl, 1H-indazol-7-yl, pyrazol-1-yl, 1H-pyrazol-3-yl, 1H-pyrazol-4-yl, 1H-pyrazol-5-yl, imidazol-1-yl, 1H-imidazol-2-yl, 1H-imidazol-4-yl, 1H-imidazol-5-yl, oxazol-2-yl, oxazol-4-yl, oxazol-5-yl, 5,6,7,8-tetrahydroisoquinolin-1-yl, 5,6,7,8-tetrahydroisoquinolin-3-yl, 5,6,7,8-tetrahydroisoquinolin-4-yl, 5,6,7,8-tetrahydroisoquinolin-5-yl, 5,6,7,8-tetrahydroisoquinolin-6-yl, 5,6,7,8-tetrahydroisoquinolin-7-yl, and 5,6,7,8-tetrahydroisoquinolin-8-yl. Particularly preferred, yet non-limiting examples of heteroaryl are 2-pyridyl, 1H-pyrazol-3-yl, and 5,6,7,8-tetrahydroisoquinolin-3-yl.

The term "hydroxy" refers to an -OH group.

The term "cyano" refers to a -CN (nitrile) group.

The term "alkylsulfonyl" refers to an alkyl group attached to the parent molecular moiety via an SO₂ moiety. A particularly preferred, yet non-limiting example of alkylsulfonyl is methylsulfonyl.

The term "haloalkyl" refers to an alkyl group, wherein at least one of the hydrogen atoms of the alkyl group has been replaced by a halogen atom, preferably fluoro. Preferably, "haloalkyl" refers to an alkyl group wherein 1, 2 or 3 hydrogen atoms of the alkyl group have been replaced by a halogen atom, most preferably fluoro. A particularly preferred, yet non-limiting example of haloalkyl is trifluoromethyl (CF₃).

The term "haloalkoxy" refers to an alkoxy group, wherein at least one of the hydrogen atoms of the alkoxy group has been replaced by a halogen atom, preferably fluoro. Preferably, "haloalkoxy" refers to an alkoxy group wherein 1, 2 or 3 hydrogen atoms of the alkoxy group have been replaced by a halogen atom, most preferably fluoro. A particularly preferred, yet non-limiting example of haloalkoxy is trifluoromethoxy (-OCF₃).

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are hydrochloride salts.

The term "protective group" (PG) denotes the group which selectively blocks a reactive site in a multifunctional compound such that a chemical reaction can be carried out selectively at another unprotected reactive site in the meaning conventionally associated with it in synthetic chemistry. Protective groups can be removed at the appropriate point. Exemplary protective groups are amino-protective groups, carboxy-protective groups or hydroxy-protective groups. Particular protective groups are the tert-butoxycarbonyl (Boc), benzyloxycarbonyl (Cbz), fluorenylmethoxycarbonyl (Fmoc) and benzyl (Bn). Further particular protective groups are the tert-butoxycarbonyl (Boc) and the fluorenylmethoxycarbonyl (Fmoc). More particular protective group is the tert-butoxycarbonyl (Boc). Exemplary protective groups and their application in organic synthesis are described, for example, in "Protective Groups in Organic Chemistry" by T. W. Greene and P. G. M. Wutts, 5th Ed., 2014, John Wiley & Sons, N.Y.

The term "urea forming reagent" refers to a chemical compound that is able to render a first amine to a species that will react with a second amine, thereby forming an urea derivative. Non-limiting examples of urea forming reagents include bis(trichloromethyl) carbonate, phosgene, trichloromethyl chloroformate, (4-nitrophenyl)carbonate and 1,1'-carbonyldiimidazole. The urea forming reagents described in G. Sartori et al., Green Chemistry 2000, 2, 140 are incorporated herein by reference.

The compounds of formula (I) can contain several asymmetric centers and can be present in the form of optically pure enantiomers, mixtures of enantiomers such as, for example, racemates, optically pure diastereioisomers, mixtures of diastereoisomers, diastereoisomeric racemates or mixtures of diastereoisomeric racemates. In a preferred embodiment, the compound of formula (I) according to the invention is a cis-enantiomer of formula (Ia) or (Ib), respectively, as described herein.

According to the Cahn-Ingold-Prelog Convention, the asymmetric carbon atom can be of the "R" or "S" configuration.

The abbreviation "MAGL" refers to the enzyme monoacylglycerol lipase. The terms "MAGL" and "monoacylglycerol lipase" are used herein interchangeably.

The term "treatment" as used herein includes: (1) inhibiting the state, disorder or condition (e.g. arresting, reducing or delaying the development of the disease, or a relapse thereof in case of maintenance treatment, of at least one clinical or subclinical symptom thereof); and/or (2) relieving the condition (i.e., causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

The term "prophylaxis" as used herein includes: preventing or delaying the appearance of clinical symptoms of the state, disorder or condition developing in a mammal and especially a human that may be afflicted with or predisposed to the state, disorder or condition but does not yet experience or display clinical or subclinical symptoms of the state, disorder or condition.

The term "neuroinflammation" as used herein relates to acute and chronic inflammation of the nervous tissue, which is the main tissue component of the two parts of the nervous system; the brain and spinal cord of the central nervous system (CNS), and the branching peripheral nerves of the peripheral nervous system (PNS). Chronic neuroinflammation is associated with neurodegenerative diseases such as Alzheimer's disease, Parkinson's disease and multiple sclerosis. Acute neuroinflammation usually follows injury to the central nervous system immediately, e.g., as a result of traumatic brain injury (TBI).

The term "traumatic brain injury" ("TBI", also known as "intracranial injury"), relates to damage to the brain resulting from external mechanical force, such as rapid acceleration or deceleration, impact, blast waves, or penetration by a projectile.

The term "neurodegenerative diseases" relates to diseases that are related to the progressive loss of structure or function of neurons, including death of neurons. Examples of neurodegenerative diseases include, but are not limited to, multiple sclerosis, Alzheimer's disease, Parkinson's disease and amyotrophic lateral sclerosis.

The term "mental disorders" (also called mental illnesses or psychiatric disorders) relates to behavioral or mental patterns that may cause suffering or a poor ability to function in life. Such features may be persistent, relapsing and remitting, or occur as a single episode. Examples of mental disorders include, but are not limited to, anxiety and depression.

The term "pain" relates to an unpleasant sensory and emotional experience associated with actual or potential tissue damage. Examples of pain include, but are not limited to, nociceptive pain, chronic pain (including idiopathic pain), neuropathic pain including chemotherapy induced neuropathy, phantom pain and phsychogenic pain. A particular example of pain is neuropathic pain, which is caused by damage or disease affecting any part of the nervous system involved in bodily feelings (i.e., the somatosensory system). In one embodiment, "pain" is neuropathic pain resulting from amputation or thoracotomy. In one embodiment, "pain" is chemotherapy induced neuropathy.

The term "neurotoxicity" relates to toxicity in the nervous system. It occurs when exposure to natural or artificial toxic substances (neurotoxins) alter the normal activity of the nervous system in such a way as to cause damage to nervous tissue. Examples of neurotoxicity include, but are not limited to, neurotoxicity resulting from exposure to substances used in chemotherapy, radiation treatment, drug therapies, drug abuse, and organ transplants, as well as exposure to heavy metals, certain foods and food additives, pesticides, industrial and/or cleaning solvents, cosmetics, and some naturally occurring substances.

The term "cancer" refers to a disease characterized by the presence of a neoplasm or tumor resulting from abnormal uncontrolled growth of cells (such cells being "cancer cells"). As used herein, the term cancer explicitly includes, but is not limited to, hepatocellular carcinoma, colon carcinogenesis and ovarian cancer.

The term "mammal" as used herein includes both humans and non-humans and includes but is not limited to humans, non-human primates, canines, felines, murines, bovines, equines, and porcines. In a particularly preferred embodiment, the term "mammal" refers to humans.

### Compounds of the Invention

In a first aspect (A1), the present invention provides compounds of Formula (I) 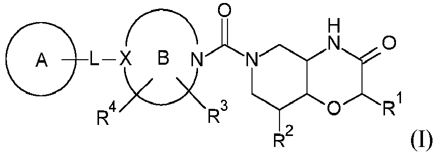 or pharmaceutically acceptable salts thereof, wherein:
- X: is N or C-R⁵;
- L: is selected from a covalent bond, -(CH₂)ₙ-O-, -O-(CH₂)ₚ-, and -SO₂-;
- n: is an integer selected from 0, 1, 2 and 3;
- p: is an integer selected from 1, 2 and 3;
- A: is:
(i) C₆₋₁₄-aryl substituted with R⁶, R⁷ and R⁸; or
(ii) 5-14 membered heteroaryl substituted with R⁹, R¹⁰ and R¹¹; or
- B: is a bridged bicyclic heterocycle;
- R¹: is hydrogen or C₁₋₆-alkyl;
- R²: is hydrogen or C₁₋₆-alkyl;
- R³: is hydrogen, C₁₋₆-alkyl, halo-C₁₋₆-alkyl, halogen or hydroxy;
- R⁴: is hydrogen, C₁₋₆-alkyl, halo-C₁₋₆-alkyl, halogen or hydroxy;
- R⁵: is hydrogen, C₁₋₆-alkyl, halo-C₁₋₆-alkyl, halogen or hydroxy; and

- R⁶, R⁷, R⁸, R⁹, R¹⁰, and R¹¹: are independently selected from hydrogen, C₁₋₆-alkyl, halo-C₁₋₆-alkyl, halogen, C₁₋₆-alkoxy, halo-C₁₋₆-alkoxy, SF₅, C₁₋₆-alkylsulfonyl, cyano, C₃₋₁₀-cycloalkyl, C₆₋₁₄-aryl, and 5-14 membered heteroaryl, wherein said C₃₋₁₀-cycloalkyl, C₆₋₁₄-aryl, and 5-14 membered heteroaryl are optionally substituted with 1-2 substituents selected from halogen, cyano, SF₅ C₁₋₆-alkyl, C₁₋₆-alkoxy, halo-C₁₋₆-alkyl, and halo-C₁₋₆-alkoxy.

Also described herein are the following enumerated Embodiments (E) of the first aspect (A1) of the invention. Only those embodiments (E) which are defined by the appended claims are included in the invention:
E1. The compound of formula (I) according to A1, or a pharmaceutically acceptable salt thereof, wherein R¹ is hydrogen.
E2. The compound of formula (I) according to A1 or E1, or a pharmaceutically acceptable salt thereof, wherein R² is hydrogen.
E3. The compound of formula (I) according to any one of A1 and E1 to E2, or a pharmaceutically acceptable salt thereof, wherein R³ is hydrogen.
E4. The compound of formula (I) according to any one of A1 and E1 to E3, or a pharmaceutically acceptable salt thereof, wherein R⁴ is hydrogen.
E5. The compound of formula (I) according to any one of A1 and E1 to E4 or a pharmaceutically acceptable salt thereof, wherein R⁵ is hydrogen.
E6. The compound of formula (I) according to any one of A1 and E1 to E5, or a pharmaceutically acceptable salt thereof, wherein R⁶ is selected from hydrogen, C₁₋₆-alkyl, halo-C₁₋₆-alkyl, and halogen.
E7. The compound of formula (I) according to any one of A1 and E1 to E5, or a pharmaceutically acceptable salt thereof, wherein R⁶ is selected from halo-C₁₋₆-alkyl and halogen.
E8. The compound of formula (I) according to any one of A1 and E1 to E5, or a pharmaceutically acceptable salt thereof, wherein R⁶ is selected from CF₃, chloro, and fluoro.
E9. The compound of formula (I) according to any one of A1 and E1 to E8, or a pharmaceutically acceptable salt thereof, wherein R⁷ is selected from hydrogen and halogen.
E10. The compound of formula (I) according to any one of A1 and E1 to E8, or a pharmaceutically acceptable salt thereof, wherein R⁷ is halogen.
E11. The compound of formula (I) according to any one of A1 and E1 to E8, or a pharmaceutically acceptable salt thereof, wherein R⁷ is fluoro or chloro.
E12. The compound of formula (I) according to any one of A1 and E1 to E8, or a pharmaceutically acceptable salt thereof, wherein R⁷ is fluoro.
E13. The compound of formula (I) according to any one of A1 and E1 to E12, or a pharmaceutically acceptable salt thereof, wherein R⁸ is hydrogen.
E14. The compound of formula (I) according to any one of A1 and E1 to E13, or a pharmaceutically acceptable salt thereof, wherein R⁹ is selected from hydrogen, C₃₋₁₀-cycloalkyl, and C₆₋₁₄-aryl, wherein said C₆₋₁₄-aryl is substituted with 1-2 substituents selected from halogen and halo-C₁₋₆-alkyl.
E15. The compound of formula (I) according to any one of A1 and E1 to E14, or a pharmaceutically acceptable salt thereof, wherein R¹⁰ is selected from hydrogen and halogen.
E16. The compound of formula (I) according to any one of A1 and E1 to E15, or a pharmaceutically acceptable salt thereof, wherein R¹¹ is hydrogen.
E17. The compound of formula (I) according to any one of A1 and E1 to E16, or a pharmaceutically acceptable salt thereof, wherein X is C-R⁵.
E18. The compound of formula (I) according to any one of A1 and E1 to E17, or a pharmaceutically acceptable salt thereof, wherein L is selected from -(CH₂)ₙ-O- and -O-(CH₂)ₚ-.
E19. The compound of formula (I) according to any one of A1 and E1 to E18, or a pharmaceutically acceptable salt thereof, wherein n is an integer selected from 0 and 1.
E20. The compound of formula (I) according to any one of A1 and E1 to E19, or a pharmaceutically acceptable salt thereof, wherein p is 1.
E21. The compound of formula (I) according to any one of A1 and E1 to E20, or a pharmaceutically acceptable salt thereof, wherein A is C₆₋₁₄-aryl substituted with R⁶, R⁷ and R⁸.
E22. The compound of formula (I) according to any one of A1 and E1 to E20, or a pharmaceutically acceptable salt thereof, wherein A is phenyl substituted with R⁶, R⁷ and R8.
E23. The compound of formula (I) according to any one of A1 and E1 to E22, or a pharmaceutically acceptable salt thereof, wherein B is a 6-14 membered bridged bicyclic heterocycle comprising 1-3 heteroatoms selected from N, S, and O.
E24. The compound of formula (I) according to any one of A1 and E1 to E22, or a pharmaceutically acceptable salt thereof, wherein B is a 6-9 membered bridged bicyclic heterocycle comprising 1-2 heteroatoms selected from N and O.
E25. The compound of formula (I) according to any one of A1 and E1 to E22, or a pharmaceutically acceptable salt thereof, wherein B is a 6-8 membered bridged bicyclic heterocycle comprising 1 nitrogen atom.
E26. The compound of formula (I) according to any one of A1 and E1 to E22, or a pharmaceutically acceptable salt thereof, wherein B is selected from 8-azabicyclo[3.2.1]octan-8-yl (a); 3,3a,4,5,6,6a-hexahydro-1H-cyclopenta[c]pyrrol-2-yl (b); 3-azabicyclo[3.1.0]hexan-3-yl (c); 2-azabicyclo[2.2.1]heptan-2-yl (d); 3-azabicyclo[3.2.1]octan-3-yl (**e**); 2,3,3a,4,6,6a-hexahydro-1H-pyrrolo[3,4-c]pyrrol-5-yl (**f**); 3-oxa-9-azabicyclo[3.3.1]nonan-9-yl (**g**); and 3-azabicyclo[3.1.1]heptan-3-yl (**h**): 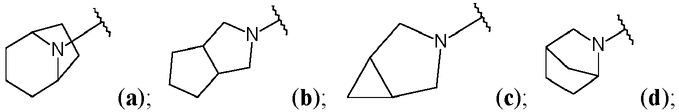 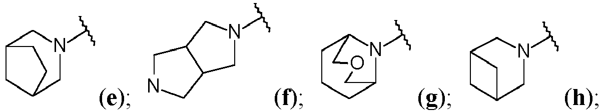 wherein a wavy line indicates the point of attachment to the carbonyl bridge bridging B to the hexahydropyrido oxazinone core of formula (I).
E27. The compound of formula (I) according to any one of A1 and E1 to E22, or a pharmaceutically acceptable salt thereof, wherein B is selected from 8-azabicyclo[3.2.1]octan-8-yl (**a**); 3,3a,4,5,6,6a-hexahydro-1H-cyclopenta[c]pyrrol-2-yl (**b**); 3-azabicyclo[3.1.0]hexan-3-yl (**c**); and 2-azabicyclo[2.2.1]heptan-2-yl (**d**): 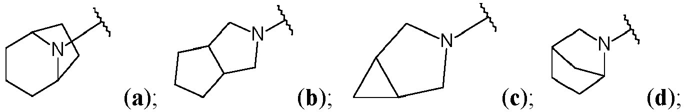 wherein a wavy line indicates the point of attachment to the carbonyl bridge bridging B to the hexahydropyrido oxazinone core of formula (I).
E28. The compound of formula (I) according to A1, or a pharmaceutically acceptable salt thereof, wherein the compound of formula (I) is a compound of formula (II): 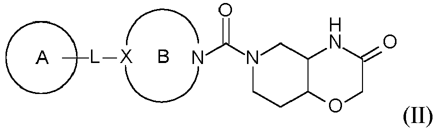 wherein:
   - X: is N or CH;
   - L: is selected from a covalent bond, -(CH₂)ₙ-O-, -OCH₂-, and -SO₂-;
   - n: is an integer selected from 0 and 1;
   - A: is:
   (i) C₆₋₁₄-aryl substituted with R⁶ and R⁷; or
   (ii) 5-14 membered heteroaryl substituted with R⁹ and R¹⁰; or
   - B: is a 6-9 membered bridged bicyclic heterocycle comprising 1-2 heteroatoms selected from N and O;
   - R⁶: is selected from hydrogen, C₁₋₆-alkyl, halo-C₁₋₆-alkyl, and halogen;
   - R⁷: is selected from hydrogen and halogen;
   - R⁹: is selected from hydrogen, C₃₋₁₀-cycloalkyl, and C₆₋₁₄-aryl, wherein said C₆₋₁₄-aryl is substituted with 1-2 substituents selected from halogen and halo-C₁₋₆-alkyl; and
   - R¹⁰: is selected from hydrogen and halogen.
E29. The compound of formula (I) according to A1, or a pharmaceutically acceptable salt thereof, wherein the compound of formula (I) is a compound of formula (III): 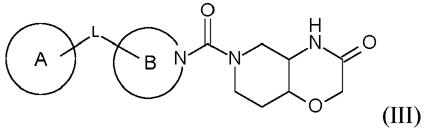 wherein:
   - L: is selected from -(CH₂)ₙ-O- and -OCH₂-;
   - n: is an integer selected from 0 and 1;
   - A: is C₆₋₁₄-aryl substituted with R⁶ and R⁷;
   - B: is a 6-8 membered bridged bicyclic heterocycle comprising 1 nitrogen atom;
   - R⁶: is selected from halo-C₁₋₆-alkyl and halogen; and
   - R⁷: is halogen.
E30. The compound of formula (I) according to A1, or a pharmaceutically acceptable salt thereof, wherein the compound of formula (I) is a compound of formula (III): 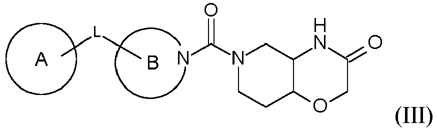 wherein:
   - L: is selected from -(CH₂)ₙ-O- and -OCH₂-;
   - n: is an integer selected from 0 and 1;
   - A: is phenyl substituted with R⁶ and R⁷;
   - B: is selected from 8-azabicyclo[3.2.1]octan-8-yl (a); 3,3a,4,5,6,6a-hexahydro-1H-cyclopenta[c]pyrrol-2-yl (b); 3-azabicyclo[3.1.0]hexan-3-yl (c); and 2-azabicyclo[2.2. 1]heptan-2-yl (d): 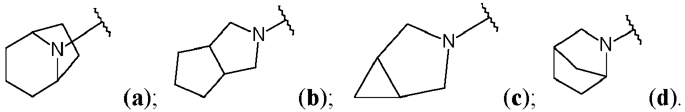
   wherein a wavy line indicates the point of attachment to the carbonyl bridge bridging B to the hexahydropyrido oxazinone core of formula (I);
   - R⁶: is selected from CF₃, chloro, and fluoro; and
   - R⁷: is fluoro or chloro.
E31. The compound of formula (I) according to any one of A1 and E1 to E30, or a pharmaceutically acceptable salt thereof, wherein said compound of formula (I) is selected from the compounds disclosed in Table 1.
E32. The compound of formula (I) according to any one of A1 and E1 to E30, or a pharmaceutically acceptable salt thereof, wherein said compound of formula (I) is selected from the group consisting of:
   (4aR,8aS)-6-[(1R,5S,6r)-6-[(2-chloro-4-fluoro-phenoxy)methyl]-3-azabicyclo[3.1.0]hexane-3-carbonyl]-4,4a,5,7,8,8a-hexahydropyrido[4,3-b][1,4]oxazin-3-one;
   (4aR,8aS)-6-[5-(2-chloro-4-fluoro-phenoxy)-3,3a,4,5,6,6a-hexahydro-1H-cyclopenta[c]pyrrole-2-carbonyl]-4,4a,5,7,8,8a-hexahydropyrido[4,3-b][1,4]oxazin-3-one;
   (4aR,8aS)-6-[rel-(1R,4R,5S)-5-[[2-fluoro-4-(trifluoromethyl)phenyl]methoxy]-2-azabicyclo[2.2.1]heptane-2-carbonyl]-4,4a,5,7,8,8a-hexahydropyrido[4,3-b][1,4]oxazin-3-one; and
   (4aR,8aS)-6-[(1S,5R)-3-[[2-fluoro-4-(trifluoromethyl)phenyl]methoxy]-8-azabicyclo[3.2.1]octane-8-carbonyl]-4,4a,5,7,8,8a-hexahydropyrido[4,3-b][1,4]oxazin-3-one.
E33. The compound of formula (I) according to any one of A1 and E1 to E30, or a pharmaceutically acceptable salt thereof, wherein R¹ and R² are both hydrogen.
E34. The compound of formula (I) according to any one of A1 and E1 to E30, or a pharmaceutically acceptable salt thereof, wherein:
   - X: is N or CH;
   - R³ and R⁴: are both hydrogen; and
   - B: is a 6-9 membered bridged bicyclic heterocycle comprising 1-2 heteroatoms selected from N and O.
E35. The compound of formula (I) according to any one of A1 and E1 to E30, or a pharmaceutically acceptable salt thereof, wherein:
   - X: is CH;
   - R³ and R⁴: are both hydrogen; and
   - B: is a 6-8 membered bridged bicyclic heterocycle comprising 1 nitrogen atom.
E36. The compound of formula (I) according to any one of A1 and E1 to E30, or a pharmaceutically acceptable salt thereof, wherein:
   - X: is CH;
   - R³ and R⁴: are both hydrogen; and
   - B: is selected from 8-azabicyclo[3.2.1]octan-8-yl (a); 3,3a,4,5,6,6a-hexahydro-1H-cyclopenta[c]pyrrol-2-yl (b); 3-azabicyclo[3.1.0]hexan-3-yl (c); and 2-azabicyclo[2.2. 1]heptan-2-yl (d): 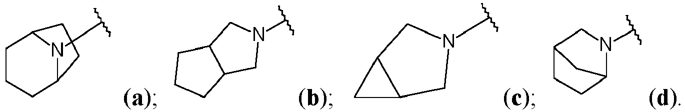
   wherein a wavy line indicates the point of attachment to the carbonyl bridge bridging B to the hexahydropyrido oxazinone core of formula (I).
E37. The compound of formula (I) according to any one of A1 and E1 to E30, or a pharmaceutically acceptable salt thereof, wherein:
   - L: is selected from a covalent bond, -(CH₂)ₙ-O-, -OCH₂-, and -SO₂-;
   - n: is an integer selected from 0 and 1;
   - A: is:
   (i) C₆₋₁₄-aryl substituted with R⁶ and R⁷; or
   (ii) 5-14 membered heteroaryl substituted with R⁹ and R¹⁰;
   - R⁶: is selected from hydrogen, C₁₋₆-alkyl, halo-C₁₋₆-alkyl, and halogen;
   - R⁷: is selected from hydrogen and halogen;
   - R⁹: is selected from hydrogen, C₃₋₁₀-cycloalkyl, and C₆₋₁₄-aryl, wherein said C₆₋₁₄-aryl is substituted with 1-2 substituents selected from halogen and halo-C₁₋₆-alkyl; and
   - R¹⁰: is selected from hydrogen and halogen.
E38. The compound of formula (I) according to any one of A1 and E1 to E30, or a pharmaceutically acceptable salt thereof, wherein:
   - L: is selected from -(CH₂)ₙ-O- and -OCH₂-;
   - n: is an integer selected from 0 and 1;
   - A: is C₆₋₁₄-aryl substituted with R⁶ and R⁷;
   - R⁶: is selected from halo-C₁₋₆-alkyl and halogen; and
   - R⁷: is halogen.
E39. The compound of formula (I) according to any one of A1 and E1 to E30, or a pharmaceutically acceptable salt thereof, wherein:
   - L: is selected from -(CH₂)ₙ-O- and -OCH₂-;
   - n: is an integer selected from 0 and 1;
   - A: is phenyl substituted with R⁶ and R⁷;
   - R⁶: is selected from CF₃, chloro, and fluoro; and
   - R⁷: is fluoro or chloro.

In a particular embodiment, the present invention provides pharmaceutically acceptable salts of the compounds according to formula (I) as described herein, especially hydrochloride salts. In a further particular embodiment, the present invention provides compounds according to formula (I) as described herein as free bases.

In some embodiments, the compounds of formula (I) are isotopically-labeled by having one or more atoms therein replaced by an atom having a different atomic mass or mass number. Such isotopically-labeled (i.e., radiolabeled) compounds of formula (I) are considered to be within the scope of this disclosure. Examples of isotopes that can be incorporated into the compounds of formula (I) include isotopes of hydrogen, carbon, nitrogen, oxygen, phosphorous, sulfur, fluorine, chlorine, and iodine, such as, but not limited to, ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Certain isotopically-labeled compounds of formula (I), for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection. For example, a compound of formula (I) can be enriched with 1, 2, 5, 10, 25, 50, 75, 90, 95, or 99 percent of a given isotope.

Substitution with heavier isotopes such as deuterium, i.e. ²H, may afford certain therapeutic advantages resulting from greater metabolic stability, for example, increased *in vivo* half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (I) can generally be prepared by conventional techniques known to those skilled in the art or by processes analogous to those described in the Examples as set out below using an appropriate isotopically-labeled reagent in place of the non-labeled reagent previously employed.

### Processes of Manufacturing

The preparation of compounds of formula (I) of the present invention may be carried out in sequential or convergent synthetic routes. Syntheses of the invention are shown in the following general schemes. The skills required for carrying out the reaction and purification of the resulting products are known to those persons skilled in the art. The substituents and indices used in the following description of the processes have the significance given herein, unless indicated to the contrary.

If one of the starting materials, intermediates or compounds of formula (I) contain one or more functional groups which are not stable or are reactive under the reaction conditions of one or more reaction steps, appropriate protective groups (as described e.g., in "Protective Groups in Organic Chemistry" by T. W. Greene and P. G. M. Wutts, 5th Ed., 2014, John Wiley & Sons, N.Y.) can be introduced before the critical step applying methods well known in the art. Such protective groups can be removed at a later stage of the synthesis using standard methods described in the literature.

If starting materials or intermediates contain stereogenic centers, compounds of formula (I) can be obtained as mixtures of diastereomers or enantiomers, which can be separated by methods well known in the art e.g., chiral HPLC, chiral SFC or chiral crystallization. Racemic compounds can e.g., be separated into their antipodes via diastereomeric salts by crystallization with optically pure acids or by separation of the antipodes by specific chromatographic methods using either a chiral adsorbent or a chiral eluent. It is equally possible to separate starting materials and intermediates containing stereogenic centers to afford diastereomerically/enantiomerically enriched starting materials and intermediates. Using such diastereomerically/enantiomerically enriched starting materials and intermediates in the synthesis of compounds of formula (I) will typically lead to the respective diastereomerically/enantiomerically enriched compounds of formula (I).

A person skilled in the art will acknowledge that in the synthesis of compounds of formula (I) - insofar not desired otherwise - an "orthogonal protection group strategy" will be applied, allowing the cleavage of several protective groups one at a time each without affecting other protective groups in the molecule. The principle of orthogonal protection is well known in the art and has also been described in literature (e.g. Barany and R. B. Merrifield, J. Am. Chem. Soc. 1977, 99, 7363; H. Waldmann et al., Angew. Chem. Int. Ed. Engl. 1996, 35, 2056).

A person skilled in the art will acknowledge that the sequence of reactions may be varied depending on reactivity and nature of the intermediates.

In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example:
Comprehensive Organic Transformations: A Guide to Functional Group Preparations, 2nd Edition, Richard C. Larock. John Wiley & Sons, New York, NY. 1999*).* It was found convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux. The time required for the reaction may also vary widely, depending on many factors, notably the reaction temperature and the nature of the reagents. However, a period of from 0.5 hours to several days will usually suffice to yield the described intermediates and compounds. The reaction sequence is not limited to the one displayed in the schemes, however, depending on the starting materials and their respective reactivity, the sequence of reaction steps can be freely altered.

If starting materials or intermediates are not commercially available or their synthesis not described in literature, they can be prepared in analogy to existing procedures for close analogues or as outlined in the experimental section.

The following abbreviations are used in the present text:
AcOH = acetic acid, ACN = acetonitrile , Bn = benzyl, Boc = tert-butyloxycarbonyl, CAS RN = chemical abstracts registration number, Cbz = benzyloxycarbonyl, Cs₂CO_{3 =} cesium carbonate, CO = carbon monoxide, CuCl = copper(I) chloride, CuCN = copper(I) cyanide, CuI = copper(I) iodide, DAST = (diethylamino)sulfur trifluoride, DBU = 1,8-diazabicyclo[5,4,0]undec-7-ene, DCM = dichloromethane, DEA = diethyl amine, DEAD = diethyl azodicarboxylate, DIAD = diisopropyl azodicarboxylate, DMAP = 4-dimethylaminopyridine, DME = dimethoxyethane , DMEDA = N,N'-dimethylethylenediamine, DMF = N,N-dimethylformamide, DIPEA = N,N-diisopropylethylamine, dppf = 1,1 bis(diphenyl phosphino)ferrocene, EDC.HCl = N-(3-dimethylaminopropyl)-N'-ethylcarbodiimide hydrochloride, EI = electron impact, ESI = electrospray ionization, EtOAc = ethyl acetate, EtOH = ethanol, h = hour(s), FA = formic acid, H₂O = water, H₂SO₄ = sulfuric acid, HATU = 1-[bis(dimethylamino)methylene]-1H-1,2,3-triazolo[4,5-b]pyridinium-3-oxide hexafluorophosphate, HBTU = O-benzotriazole-N,N,N',N'-tetramethyl-uronium-hexafluoro-phosphate, HCl = hydrogen chloride, HOBt = 1-hydroxy-1H-benzotriazole; HPLC = high performance liquid chromatography, iPrMgCl = isopropylmagnesium chloride, I₂ = iodine, IPA = 2-propanol, ISP = ion spray positive (mode), ISN = ion spray negative (mode), K₂CO₃ = potassium carbonate, KHCO₃ = potassium bicarbonate, KI = potassium iodide, KOH = potassium hydroxide, K₃PO₄ = potassium phosphate tribasic, LiAlH₄ or LAH = lithium aluminium hydride, LiHMDS = lithium bis(trimethylsilyl)amide, LiOH = lithium hydroxide, MgSO₄ = magnesium sulfate, min = minute(s), mL = milliliter, MPLC = medium pressure liquid chromatography, MS = mass spectrum, nBuLi = n-butyllithium, NaBH₃CN = sodium cyanoborohydride, NaH = sodium hydride, NaHCO₃ = sodium hydrogen carbonate, NaNO₂ = sodium nitrite, NaBH(OAc)₃ = sodium triacetoxyborohydride, NaOH = sodium hydroxide, Na₂CO₃ = sodium carbonate, Na₂SO₄ = sodium sulfate, Na₂S₂O₃ = sodium thiosulfate, NBS = N-bromosuccinimide, nBuLi = n-butyllithium, NEt₃ = triethylamine (TEA), NH₄Cl = ammonium chloride, NMP = N-methyl-2-pyrrolidone, OAc = Acetoxy, T₃P = propylphosphonic anhydride, PE = petroleum ether, PG = protective group, Pd-C = palladium on activated carbon, PdCl₂(dppf)-CH₂Cl₂ = 1,1'-bis(diphenylphosphino)ferrocene-palladium(II)dichloride dichloromethane complex, Pd₂(dba)₃ = tris(dibenzylideneacetone)dipalladium(0), Pd(OAc)₂ = palladium(II) acetate, Pd(OH)₂ = palladium hydroxide, Pd(PPh₃)₄ = tetrakis(triphenylphosphine)palladium(0), PTSA = p-toluenesulfonic acid, R = any group, RT = room temperature, SFC = Supercritical Fluid Chromatography, S-PHOS = 2-dicyclohexylphosphino-2',6'-dimethoxybiphenyl, TBAI = tetra butyl ammonium iodine, TEA = triethylamine, TFA = trifluoroacetic acid, THF = tetrahydrofuran, TMEDA = N,N,N',N'-tetramethylethylenediamine, ZnCl₂ = zinc chloride, Hal = halogen.

Compounds of formula I wherein A, L, X, R¹, R², R³ and R⁴ are as described herein can be synthesized in analogy to literature procedures and/or as depicted for example in Scheme 1.

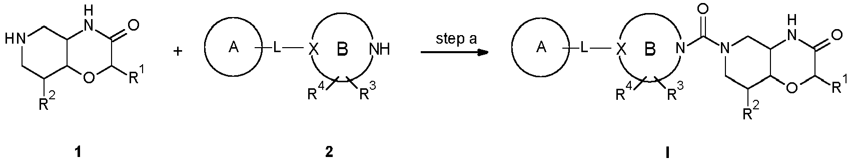

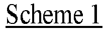

Accordingly, 4a,5,6,7,8,8a-hexahydro-4H-pyrido[4,3-b][1,4]oxazin-3-ones 1 are reacted with intermediates 2 in the presence of a urea forming reagent such as bis(trichloromethyl) carbonate using a suitable base and solvent such as, e.g. sodium bicarbonate in DCM, to give compounds of formula I (step a). Further urea forming reagents include but are not limited to phosgene, trichloromethyl chloroformate, (4-nitrophenyl)carbonate, 1,1'-carbonyldiimidazole or 1,1'-carbonyl-di-(1,2,4-triazole). Reactions of this type and the use of these reagents are widely described in literature (e.g. G. Sartori et al., Green Chemistry 2000, 2, 140). A person skilled in the art will acknowledge that the order of the addition of the reagents can be important in this type of reactions due to the reactivity and stability of the intermediary formed carbamoyl chlorides, as well as for avoiding formation of undesired symmetrical urea by-products.

Intermediates 1 may be synthesized as depicted for example in Scheme 2 and/or in analogy to methods described in literature.

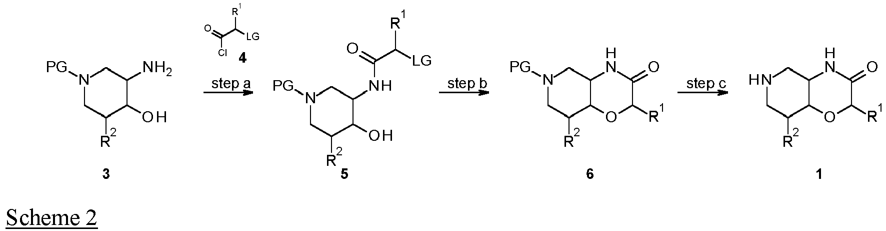

Thus, 3-aminopiperidin-4-ol derivatives 3 in which "PG" signifies a suitable protective group such as a Cbz or Boc protective group, and R² is as defined herein can be acylated for example with acyl chlorides **4** in which R¹ is as defined herein and "LG" signifies a suitable leaving group (e.g., Cl or Br), using a suitable base such as sodium or potassium carbonate, sodium hydroxide or sodium acetate in an appropriate solvent such as THF, water, acetone or mixtures thereof, to provide intermediates **5** (step a). Intermediates **4** are either commercially available or can be prepared according to literature methods in achiral (R¹ = H) racemic (R¹ not H) or enantiomerically pure form (R¹ not H).

Intermediates **5** can be cyclized to intermediates **6** using methods well known in the art, for example by treatment of 5 with sodium hydride in THF or potassium tert-butoxide in IPA and water (step b). Reactions of that type are described in literature (e.g. Z. Rafinski et al., J. Org. Chem. 2015, 80, 7468; S. Dugar et al., Synthesis 2015, 47(5), 712; WO2005/066187). Removal of the protective group in intermediates 6, applying methods known in the art (e.g., a Boc group using TFA in DCM at temperatures between 0°C and room temperature, a Cbz group using hydrogen in the presence of a suitable catalyst such as Pd or Pd(OH)₂ on charcoal in a suitable solvent such as MeOH, EtOH, EtOAc or mixtures thereof and as described for example in "Protective Groups in Organic Chemistry" by T.W. Greene and P.G.M. Wuts, 4th Ed., 2006, Wiley N.Y.), furnishes intermediates 1 (step c).

Intermediates 1 can be obtained as mixtures of diastereomers and enantiomers, respectively, or as single stereoisomers depending on whether racemic mixtures or enantiomerically pure forms of cis- or trans-3-aminopiperidin-4-ol derivatives **3** and acid chlorides **4** (when R¹ is not H) are employed in their syntheses. In case racemization occurs at a stereocentre bearing R¹ during the conversion of **3** to **5** (step a) and/or of **5** to **6** (step b), the resulting diastereoisomers may be separated by chromatography (e.g. HPLC, chiral HPLC) or other methods known in the art. Intermediates **3** are commercially available and their synthesis has also been described in literature (e.g. WO2005/066187; WO2011/0059118; WO2016/185279). Optically pure cis-configured intermediates **1B** and **1C** can be obtained for example according to Scheme 3 by chiral separation of commercially available rac-(4aR,8aS)-4a,5,6,7,8,8a-hexahydro-4H-pyrido[4,3-b][1,4]oxazin-3-one (**1A**) (optionally in form of a salt such as, e.g. a hydrochloride salt) using methods known in the art, e.g. by diastereomeric salt crystallization or by chiral chromatography (step a).

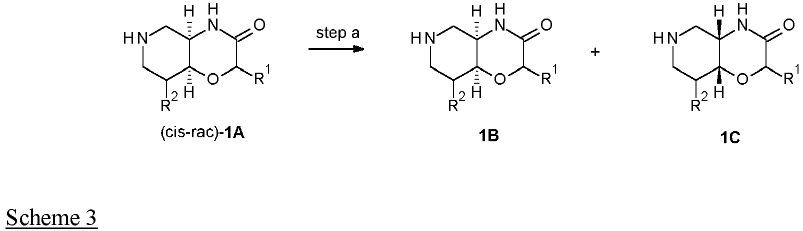

In some embodiments, intermediates **2** are intermediates of type **A.** Intermediates of type **A** in which A, B, R³ and R⁴ are as described herein and R⁵ is hydrogen, C₁₋₆-alkyl or halo-C₁₋₆-alkyl, can be prepared by methods well known by a person skilled in the art and as exemplified by the general synthetic procedure outlined in Scheme 4.

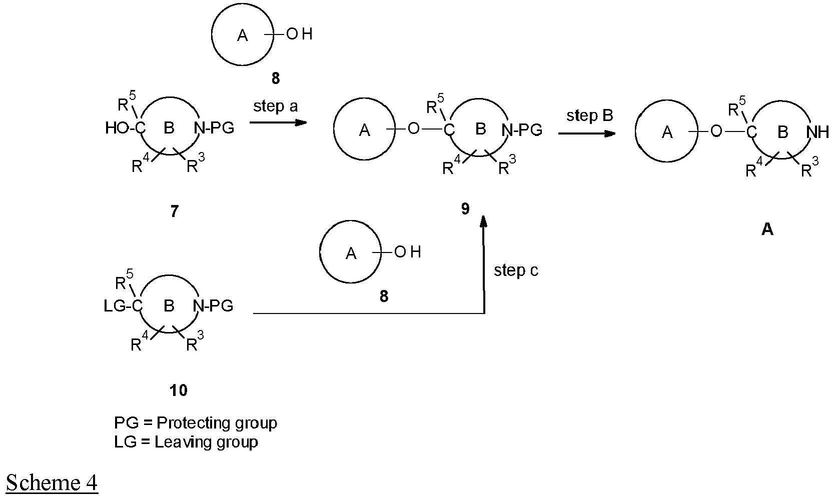

Bicyclic compounds 7 in which PG signifies a suitable protective group such as a Boc, Cbz or Bn protecting group (either commercially available or prepared as described in literature, e.g. in Eur. J. Org. Chem. 2017, 36, 5316; Topics in Het. Chem. 2014, 35, 189; World Journal of Pharmacy and Pharmaceutical Sciences 2014, 3(12), 536; Chem. Rev. 2014, 114(16), 8257-8322) can be subjected to a Mitsunobu reaction with alcohol derivatives 8 using an appropriate phosphine such as triphenylphosphine and a dialkyl azodicarboxylate such as DEAD or DIAD in a suitable solvent such as THF to give intermediates 9 (step a). Mitsunobu reactions of that type are broadly described in literature (e.g. Org. Chem. Front. 2015, 2, 739; Chem. Rev. 2009, 109 (6), 2551).

Removal of the protective group from intermediates 9, applying methods known in the art, e.g., a Boc group using TFA in DCM or 4M HCl in dioxane at temperatures between 0°C and room temperature, a Bn or Cbz group using hydrogen in the presence of a suitable catalyst such as Pd or Pd(OH)₂ on charcoal in a suitable solvent such as MeOH, EtOH, EtOAc or mixtures thereof and as described for example in "Protective Groups in Organic Chemistry" by T.W. Greene and P.G.M. Wuts, 4th Ed., 2006, Wiley N.Y.), furnishes intermediates **A** (step b).

Intermediates **9** may alternatively be prepared by alkylation of compounds **8** with spirocyclic derivatives **10** (either commercially available or prepared by methods known in the art) in which LG signifies a suitable leaving group such as chlorine, bromine, iodine, OSO₂alkyl (e.g. mesylate (methanesulfonate), OSO₂fluoroalkyl (e.g. triflate (trifluoromethanesulfonate) or OSO₂aryl (e.g. tosylate (p-toluenesulfonate) using a suitable base and an appropriate solvent (e.g. sodium hydride in DMF or potassium tert-butoxide in THF) at temperatures between 0°C and the boiling temperature of the solvent (step c).

In some embodiments, intermediates **2** are intermediates of type **B.** Intermediates of type **B** in which A, B, R³ and R⁴ are as described herein and R⁵ is hydrogen; C₁₋₆-alkyl or halo-C₁₋₆-alkyl can be prepared by methods well known in the art and as exemplified by the general synthetic procedures outlined in Scheme 5.

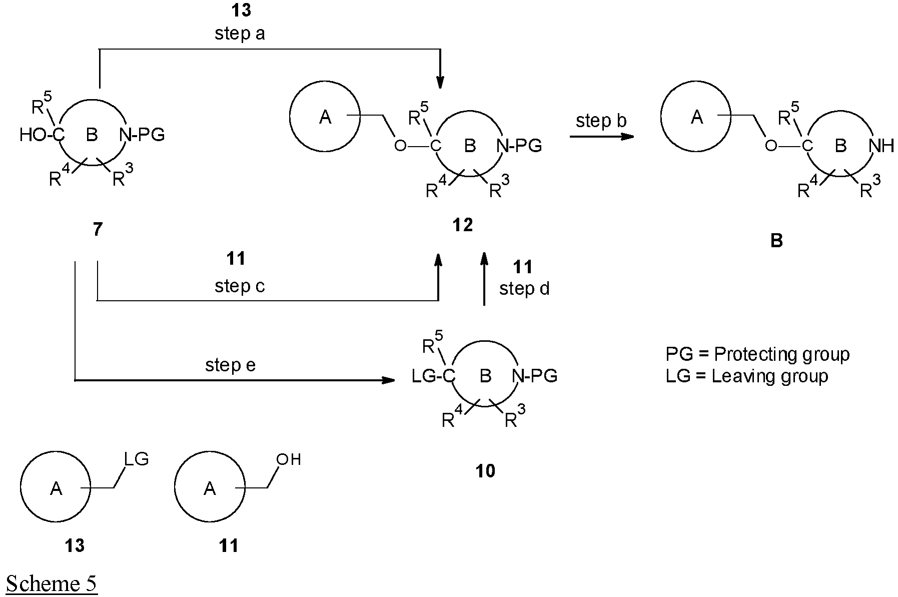

Bicyclic compounds **7** in which PG is a suitable protective group can be alkylated with compounds **13** in which LG is a suitable leaving group such as chlorine, bromine, iodine, methanesulfonate, trifluoromethanesulfonate or p-toluenesulfonate (prepared by literature methods for example from compounds **11**) using using a suitable base and an appropriate solvent (e.g. sodium hydride in DMF or potassium tert-butoxide in THF) at temperatures between 0°C and the boiling temperature of the solvent (step c) to provide intermediates **12** (step a).

Removal of the protective group from intermediates **12**, applying methods known in the art and for example described under Scheme 4, step b, furnishes intermediates **B** (step b).

Alternatively, intermediates **12** may be prepared from intermediates **7** and compounds **11** via Mitsunobu reaction, applying for example the conditions described under Scheme 4, step a (step c).

Furthermore, intermediates **12** may be also prepared by alkylation of compounds **7** with compounds **10** and using for example the conditions described under Scheme 4, step c (step d). Intermediates **10** in turn may be synthesized from compounds **7** converting the hydroxy function into a suitable leaving group such as an alkyl halide (e.g. bromine by using of PBr₃, chlorine through the use of SOCl₂) or alkyl- or aryl-sulfonate such as methanesulfonate (using mesyl chloride) or p-toluenesulfonate (using tosyl chloride). Reactions of that type are broadly described in literature and are well known in the art.

In some embodiments, intermediates **2** are intermediates of type **C.** Intermediates of type **C** in which A is aryl, B, R³ and R⁴, R¹², R¹³ are as described herein and R⁵ is hydrogen; C₁₋₆-alkyl or halo-C₁₋₆-alkyl can be prepared by methods well known in the art and as exemplified by the general synthetic procedures outlined in Scheme 6 and described in more detail in J. Med. Chem. 2018, 61, 3008-3026.

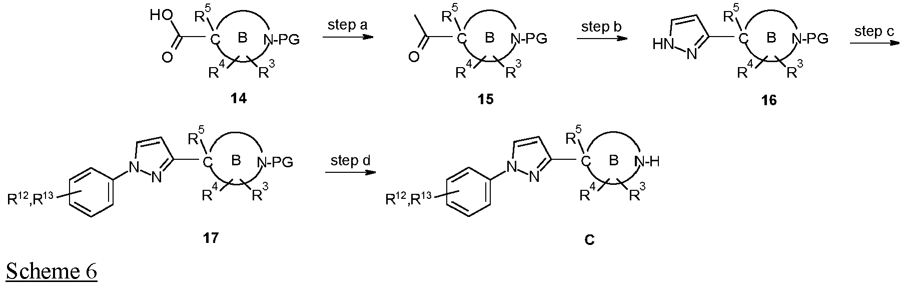

Also disclosed herein, but not part of the invention, is a process of manufacturing the urea compounds of formula (I) described herein, and pharmaceutically acceptable salts thereof, comprising:
(a) reacting a first amine of formula 1, wherein R¹ and R² are as described herein, preferably wherein R¹ and R² are hydrogen, 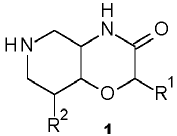 with a second amine **2**, wherein A, B. L. X, R³ and R⁴ are as described herein 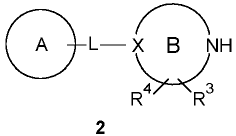 in the presence of a base and a urea forming reagent, to form said compound of formula (I); and optionally
(b) transforming said compound of formula (I) to a pharmaceutically acceptable salts thereof.

In one embodiment, there is provided a process according to the invention, wherein said base is sodium bicarbonate.

In one embodiment, there is provided a process according to the invention, wherein said urea forming reagent is selected from bis(trichloromethyl) carbonate, phosgene, trichloromethyl chloroformate, (4-nitrophenyl)carbonate and 1,1'-carbonyldiimidazole, preferably wherein said urea forming reagent is bis(trichloromethyl) carbonate.

### MAGL Inhibitory Activity

Compounds of the present invention are MAGL inhibitors. Thus, in one aspect, the present invention provides the compounds of formula (I) as described herein for use in inhibiting

### MAGL in a mammal.

In a further aspect, the present invention provides compounds of formula (I) as described herein for use in a method of inhibiting MAGL in a mammal.

In a further aspect, the present invention provides the compounds of formula (I) as described herein for use in a method for inhibiting MAGL in a mammal, which method comprises administering an effective amount of a compound of formula (I) as described herein to the mammal.

Compounds were profiled for MAGL inhibitory activity by determining the enzymatic activity by following the hydrolysis of the natural substrate 2-arachidonoylglycerol resulting in arachidonic acid, which can be followed by mass spectrometry. This assay is hereinafter abbreviated "2-AG assay".

The 2-AG assay was carried out in 384 well assay plates (PP, Greiner Cat# 784201) in a total volume of 20 µL. Compound dilutions were made in 100% DMSO (VWR Chemicals 23500.297) in a polypropylene plate in 3-fold dilution steps to give a final concentration range in the assay from 12.5 µM to 0.8 pM. 0.25µL compound dilutions (100% DMSO) were added to 9 µL MAGL in assay buffer (50 mM TRIS (GIBCO, 15567-027), 1 mM EDTA (Fluka, 03690-100ml), 0.01% (v/v) Tween. After shaking, the plate was incubated for 15 min at RT. To start the reaction, 10 µL 2-arachidonoylglycerol in assay buffer was added. The final concentrations in the assay was 50 pM MAGL and 8 µM 2-arachidonoylglyerol. After shaking and 30 min incubation at RT, the reaction was quenched by the addition of 40µL of acetonitrile containing 4µM of d8-arachidonic acid. The amount of arachidonic acid was traced by an online SPE system (Agilent Rapidfire) coupled to a triple quadrupole mass spectrometer (Agilent 6460). A C18 SPE cartridge (G9205A) was used in an acetonitrile/water liquid setup. The mass spectrometer was operated in negative electrospray mode following the mass transitions 303.1 → 259.1 for arachidonic acid and 311.1 → 267.0 for d8-arachidonic acid. The activity of the compounds was calculated based on the ratio of intensities [arachidonic acid / d8-arachidonic acid].

**Table 1**

| **Ex.** | Structure | **IUPAC Name** | **MAGL IC50 (nM)** |
|---|---|---|---|
| **1** | 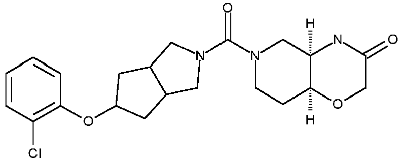 | (4aR,8aS)-6-(5-(2-chlorophenoxy)-3,3a,4,5,6,6a-hexahydro-1H-cyclopenta[c]pyrrole-2-carbonyl)-4,4a,5,7,8,8a-hexahydropyrido[4,3-b][1,4]oxazin-3-one | **8.9** |
| **2** | 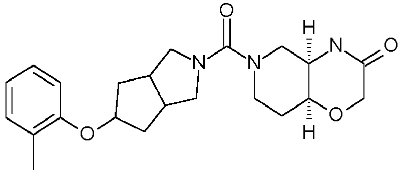 | (4aR,8aS)-6-(5-(2-methylphenoxy)-3,3a,4,5,6,6a-hexahydro-1H-cyclopenta[c]pyrrole-2-carbonyl)-4,4a,5,7,8,8a-hexahydropyrido[4,3-b][1,4]oxazin-3-one | **19.8** |
| **3** | 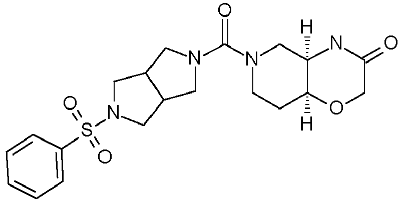 | (4aR,8aS)-6-(5-(benzenesulfonyl)-1,3,3a,4,6,6a-hexahydropyrrolo[3,4-c]pyrrole-2-carbonyl)-4,4a, 5,7, 8, 8a-hexahydropyrido [4,3-b][1,4]oxazin-3-one | **2278.6** |
| **4** | 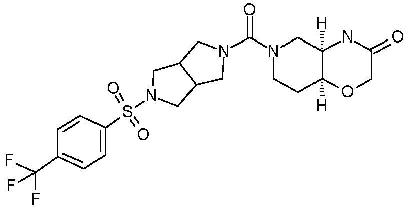 | (4aR,8aS)-6-(5-[4-(trifluoromethyl)phenyl]sulfonyl-1,3,3a,4,6,6a-hexahydropyrrolo[3,4-c]pyrrole-2-carbonyl)-4,4a, 5,7, 8, 8a-hexahydropyrido [4,3-b][1,4]oxazin-3-one | **929.2** |
| **5** | 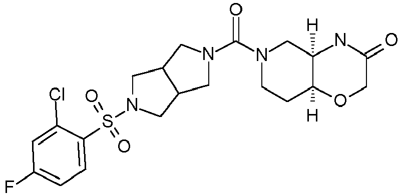 | (4aR,8aS)-6-(5-(2-chloro-4-fluorophenyl)sulfonyl-1,3,3a,4,6,6a-hexahydropyrrolo[3,4-c]pyrrole-2-carbonyl)-4,4a, 5,7, 8, 8a-hexahydropyrido [4,3-b][1,4]oxazin-3-one | **675.6** |
| **6** | 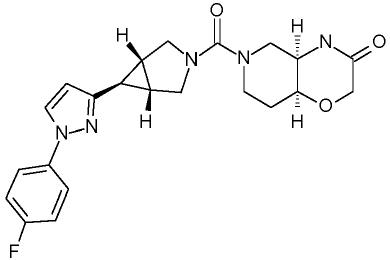 | (4aR,8aS)-6-[(1R,5S,6r)-6-[1-(4-fluorophenyl)pyrazol-3-yl]-3 - azabicyclo[3. 1.0]hexane-3-carbonyl]-4,4a,5,7,8,8a-hexahydropyrido [4,3-b][1,4]oxazin-3-one | **260.6** |
| **7** | 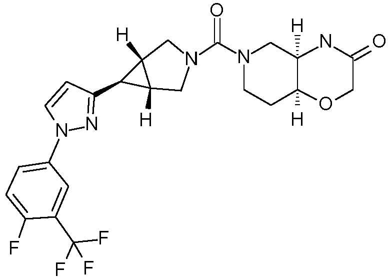 | (4aR,8aS)-6-[(1R,5S,6r)-6-[1-[4-fluoro-3-(trifluoromethyl)phenyl]pyrazol-3 - yl]-3-azabicyclo[3.1.0]hexane-3-carbonyl]-4,4a,5,7,8,8a-hexahydropyrido [4,3-b][1,4]oxazin-3-one | **45.3** |
| **8** | 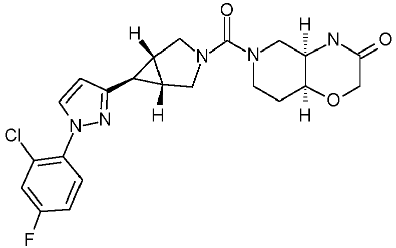 | (4aR,8aS)-6-[(1R,5S,6r)-6-[1-(2-chloro-4-fluoro-phenyl)pyrazol-3 - yl]-3-azabicyclo[3.1.0]hexane-3-carbonyl]-4,4a,5,7,8,8a-hexahydropyrido[4,3-b][1,4]oxazin-3-one | **111.7** |
| **9** | 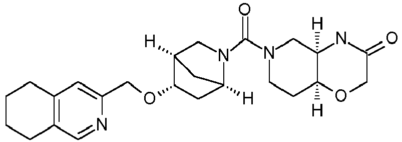 | (4aR,8aS)-6-[rel-(1R,4R,5S)-5-(5,6,7,8-tetrahydroisoquinolin-3-ylmethoxy)-2-azabicyclo[2.2.1]heptane-2-carbonyl]-4,4a,5,7,8,8a-hexahydropyrido[4,3-b][1,4]oxazin-3-one | **76.0** |
| **10** | 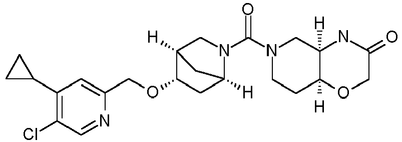 | (4aR, 8aS) -6- [rel- (lR,4R, 5 S) -5 - [(5 - chloro-4-cyclopropyl-2-pyridyl)methoxy]-2-azabicyclo[2.2.1]heptane-2-carbonyl]-4,4a,5,7,8,8a-hexahydropyrido [4,3-b][1,4]oxazin-3-one | **16.2** |
| **11** | 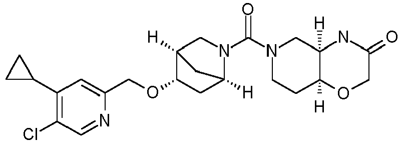 | (4aR, 8aS)-6- [rel-(lR,4R,5 S)-5- [(5-chloro-4-cyclopropyl-2-pyridyl)methoxy]-2-azabicyclo[2.2.1]heptane-2-carbonyl]-4,4a,5,7,8,8a-hexahydropyrido [4,3-b][1,4]oxazin-3-one | **82.5** |
| **12** | 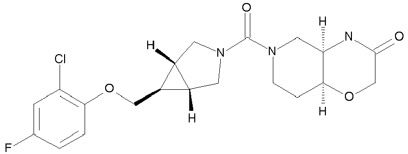 | (4aR,8aS)-6-[(1R,5S,6r)-6-[(2-chloro-4-fluoro-phenoxy)methyl]-3-azabicyclo[3.1.0]hexane-3-carbonyl]-4,4a,5,7,8,8a-hexahydropyrido [4,3-b][1,4]oxazin-3-one | **6.3** |
| **13** | 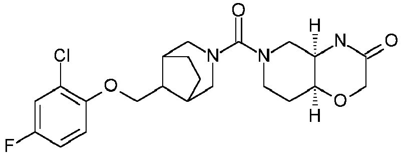 | (4aR,8aS)-6-[8-[(2-chloro-4-fluoro-phenoxy)methyl]-3-azabicyclo[3.2.1]octane-3-carbonyl]-4,4a,5,7,8,8a-hexahydropyrido[4,3-b][1,4]oxazin-3-one | **5.1** |
| **14** | 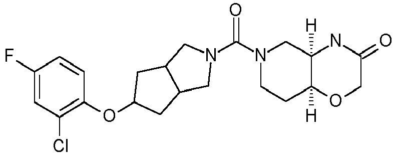 | (4aR,8aS)-6-[5-(2-chloro-4-fluoro-phenoxy)-3,3a,4,5,6,6a-hexahydro-1H-cyclopenta[c]pyrrole-2-carbonyl] -4,4a,5,7,8,8a-hexahydropyrido [4,3-b][1,4]oxazin-3-one | **3.5** |
| **15** | 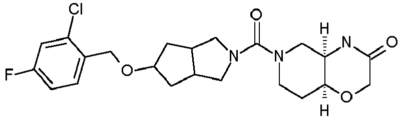 | (4aR,8aS)-6-[5-[(2-chloro-4-fluoro-phenyl)methoxy]-3,3a,4,5,6,6a-hexahydro-1H-cyclopenta [c]pyrrole-2-carbonyl]-4,4a,5,7,8,8a-hexahydropyrido[4,3-b][1,4]oxazin-3-one | **11.0** |
| **16** | 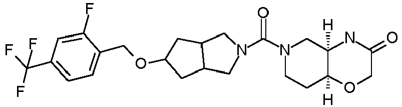 | (4aR,8aS)-6-[5-[[2-fluoro-4-(trifluoromethyl)phenyl]methoxy]-3,3a,4,5,6,6a-hexahydro-1H-cyclopenta [c]pyrrole-2-carbonyl]-4,4a,5,7,8,8a-hexahydropyrido[4,3-b][1,4]oxazin-3-one | **85.9** |
| **17** | 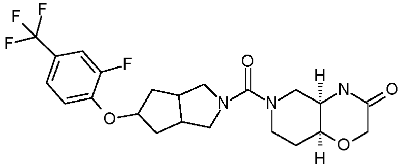 | (4aR,8aS)-6-[5-[2-fluoro-4-(trifluoromethyl)phenoxy] - 3,3a,4,5,6,6a-hexahydro-1H-cyclopenta [c]pyrrole-2-carbonyl]-4,4a,5,7,8,8a-hexahydropyirido[4,3-b][1,4]oxazin-3-one | **4.7** |
| **18** | 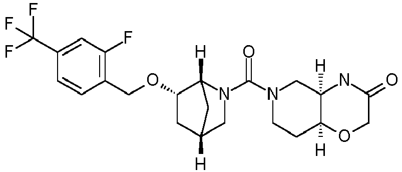 | (4aR,8aS)-6-[rel-(1R,4S,6S)-6-[[2-fluoro-4-(trifluoromethyl)phenyl]methoxy] - 2-azabicyclo[2.2.1]heptane-2-carbonyl]-4,4a,5,7,8,8a-hexahydropyrido[4,3-b][1,4]oxazin-3-one | **1049.4** |
| **19** | 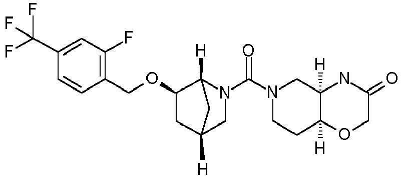 | (4aR,8aS)-6-[rel-(1R,4S,6R)-6-[[2-fluoro-4-(trifluoromethyl)phenyl]methoxy] - 2-azabicyclo[2.2.1]heptane-2-carbonyl]-4,4a,5,7,8,8a-hexahydropyrido[4,3-b][1,4]oxazin-3-one | **1142.2** |
| **20** | 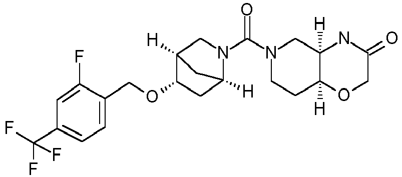 | (4aR,8aS)-6-[rel-(1R,4R,5S)-5-[[2-fluoro-4-(trifluoromethyl)phenyl]methoxy] - 2-azabicyclo[2.2.1]heptane-2-carbonyl]-4,4a,5,7,8,8a-hexahydropyrido[4,3-b][1,4]oxazin-3-one | **9.7** |
| **21** | 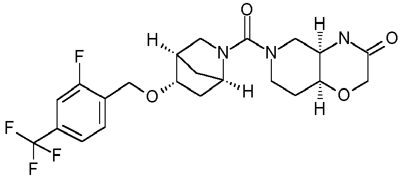 | (4aR,8aS)-6-[rel-(1R,4R,5S)-5-[[2-fluoro-4-(trifluoromethyl)phenyl]methoxy] - 2-azabicyclo[2.2.1]heptane-2-carbonyl]-4,4a,5,7,8,8a-hexahydropyrido [4,3-b][1,4]oxazin-3-one | **18.6** |
| **22** | 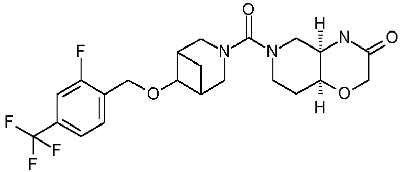 | (4aR,8aS)-6-[6-[[2-fluoro-4-(trifluoromethyl)phenyl]methoxy] - 3-azabicyclo[3.1.1]heptane-3-carbonyl]-4,4a,5,7,8,8a-hexahydropyrido [4,3-b][1,4]oxazin-3-one | **217.3** |
| **23** | 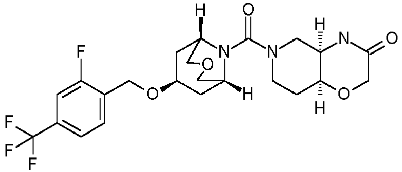 | (4aR,8aS)-6-[(1S,5R)-7-[[2-fluoro-4-(trifluoromethyl)phenyl]methoxy] - 3-oxa-9-azabicyclo[3.3. 1]nonane-9-carbonyl]-4,4a,5,7,8,8a-hexahydropyrido [4,3-b][1,4]oxazin-3-one | **628.5** |
| **24** | 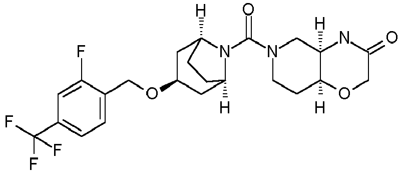 | (4aR,8aS)-6-[(1S,5R)-3-[[2-fluoro-4-(trifluoromethyl)phenyl]methoxy] - 8-azabicyclo[3.2.1]octane-8-carbonyl]-4,4a,5,7,8,8a-hexahydropyrido[4,3-b][1,4]oxazin-3-one | **1.4** |
| **25** | 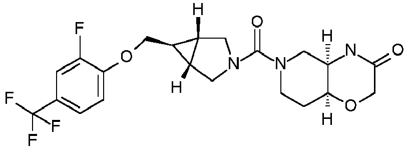 | (4aR,8aS)-6-[(1R,5S,6r)-6-[[2-fluoro-4-(trifluoromethyl)phenoxy]methyl]-3-azabicyclo[3.1.0]hexane-3-carbonyl]-4,4a,5,7,8,8a-hexahydropyrido[4,3-b][1,4]oxazin-3-one | **32.0** |
| **26** | 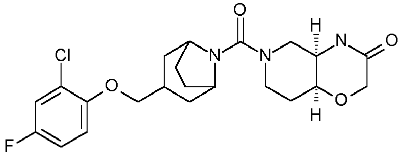 | (4aR,8aS)-6-[3-[(2-chloro-4-fluoro-phenoxy)methyl]-8-azabicyclo[3.2.1]octane-8-carbonyl]-4,4a,5,7,8,8a-hexahydropyrido [4,3-b][1,4]oxazin-3-one | **3694.0** |
| **27** | 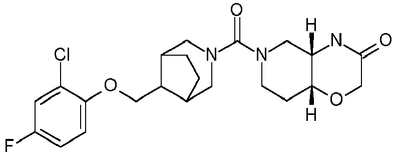 | (4aS,8aR)-6-[8-[(2-chloro-4-fluoro-phenoxy)methyl]-3-azabicyclo[3.2.1]octane-3-carbonyl]-4,4a,5,7,8,8a-hexahydropyrido[4,3-b][1,4]oxazin-3-one | **669.7** |
| **28** | 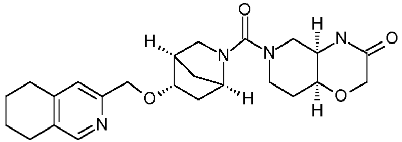 | (4aR,8aS)-6-[rel-(1R,4R,5S)-5-(5,6,7,8-tetrahydroisoquinolin-3-ylmethoxy)-2-azabicyclo[2.2.1]heptane-2-carbonyl]-4,4a,5,7,8,8a-hexahydropyrido[4,3-b][1,4]oxazin-3-one | **54.5** |

In one aspect, the present invention provides compounds of formula (I) and their pharmaceutically acceptable salts or esters as described herein, wherein said compounds of formula (I) and their pharmaceutically acceptable salts or esters have IC₅₀'s for MAGL inhibition below 25 µM, preferably below 10 µM, more preferably below 5 µM as measured in the MAGL assays described herein.

In one embodiment, compounds of formula (I) and their pharmaceutically acceptable salts or esters as described herein have IC₅₀ (MAGL inhibition) values between 0.000001 µM and 25 µM, particular compounds have IC₅₀ values between 0.000005 µM and 10 µM, further particular compounds have IC₅₀ values between 0.00005 µM and 5 µM, as measured in the MAGL assays described herein.

### Using the Compounds of the Invention

In one aspect, the present invention provides compounds of formula (I) as described herein for use as therapeutically active substance.

In one aspect, the present invention provides compounds of formula (I) as described herein for use in the treatment or prophylaxis of neuroinflammation, neurodegenerative diseases, pain, cancer, mental disorders and/or inflammatory bowel disease in a mammal.

In one embodiment, the present invention provides compounds of formula (I) as described herein for use in the treatment or prophylaxis of neuroinflammation and/or neurodegenerative diseases in a mammal.

In one embodiment, the present invention provides compounds of formula (I) as described herein for use in the treatment or prophylaxis of cancer in a mammal.

In one embodiment, the present invention provides compounds of formula (I) as described herein for use in the treatment or prophylaxis of neurodegenerative diseases in a mammal.

In one embodiment, the present invention provides compounds of formula (I) as described herein for use in the treatment or prophylaxis of inflammatory bowel disease in a mammal.

In one embodiment, the present invention provides compounds of formula (I) as described herein for use in the treatment or prophylaxis of pain in a mammal.

In one aspect, the present invention provides compounds of formula (I) as described herein for use in the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain, spasticity associated with pain, abdominal pain, abdominal pain associated with irritable bowel syndrome and/or visceral pain in a mammal.

In a preferred embodiment, the present invention provides compounds of formula (I) as described herein for use in the treatment or prophylaxis of multiple sclerosis, Alzheimer's disease and/or Parkinson's disease in a mammal.

In a particularly preferred embodiment, the present invention provides compounds of formula (I) as described herein for use in the treatment or prophylaxis of multiple sclerosis in a mammal.

### Pharmaceutical Compositions and Administration

In one aspect, the present invention provides a pharmaceutical composition comprising a compound of formula (I) as described herein and a therapeutically inert carrier.

Exemplary, yet non-limiting examples of possible pharmaceutical compositions comprising compounds of formula (I) are described in Examples 29 and 30.

The compounds of formula (I) and their pharmaceutically acceptable salts and esters can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays) or rectally (e.g. in the form of suppositories). However, the administration can also be effected parentally, such as intramuscularly or intravenously (e.g. in the form of injection solutions).

The compounds of formula (I) and their pharmaceutically acceptable salts and esters can be processed with pharmaceutically inert, inorganic or organic adjuvants for the production of tablets, coated tablets, dragées and hard gelatin capsules. Lactose, corn starch or derivatives thereof, talc, stearic acid or its salts etc. can be used, for example, as such adjuvants for tablets, dragées and hard gelatin capsules.

Suitable adjuvants for soft gelatin capsules are, for example, vegetable oils, waxes, fats, semisolid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semisolid or liquid polyols, etc.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should be appropriate. It will, however, be clear that the upper limit given herein can be exceeded when this is shown to be indicated.

### Examples

The invention will be more fully understood by reference to the following examples.

In case the preparative examples are obtained as a mixture of enantiomers, the pure enantiomers can be separated by methods described herein or by methods known to the man skilled in the art, such as e.g., chiral chromatography (e.g., chiral SFC) or crystallization.

All reaction examples and intermediates were prepared under an argon atmosphere if not specified otherwise.

### General Methods:

### At (illustrated for example 3)

To an ice-cold solution of bis(trichloromethyl) carbonate (28.8 mg, 97.1 µmol) in DCM (1.83 ml) were added sodium bicarbonate (46.6 mg, 555 µmol) and (4aR,8aS)-hexahydro-2H-pyrido[4,3-b][1,4]oxazin-3(4H)-one acetate (**BB1**, 30 mg, 139 µmol) and the mixture was stirred overnight at room temperature. To the suspension was added (3aR,6aS)-2-(phenylsulfonyl)octahydropyrrolo[3,4-c]pyrrole hydrochloride (**BB5**, 40.1 mg, 139 µmol) and DIPEA (71.7 mg, 96.9 µl, 555 µmol). The suspension was stirred at RT for 1.5 hours. The reaction mixture was poured on water and DCM and the layers were separated. The aqueous layer was extracted three times with DCM. The organic layers were washed twice with water, dried over MgSO₄, filtered, treated with silica gel and evaporated. The compound was purified by prep HPLC (Method B3) to get the desired compound as a white solid (44 mg; 73%). LC-MS (ESI): [M+H⁺]⁺ = 435.2 m/z

### A2 (illustrated for example 1)

5-(2-chlorophenoxy)octahydrocyclopenta[c]pyrrole HCl (CAS 1889054-91-4, 45 mg, 164 µmol) was dissolved in acetonitrile (0.5 ml). 4-nitrophenyl (4aR,8aS)-3-oxohexahydro-2H-pyrido[4,3-b][1,4]oxazine-6(5H)-carboxylate (**BB2**, 52.7 mg, 164 µmol) and DIPEA (63.6 mg, 86 µl, 492 µmol) were added at room temperature, followed by stirring at 80°C for 20h. The reaction mixture was concentrated *in vacuo* and purified by reverse phase HPLC (method B1). The purified product was concentrated *in vacuo* to afford (4aR,8aS)-6-(5-(2-chlorophenoxy)octahydrocyclopenta[c]pyrrole-2-carbonyl)hexahydro-2H-pyrido[4,3-b][1,4]oxazin-3(4H)-one (5 mg, 7.26 % yield) as a colorless solid. LC-MS (ESI): [M+H⁺]⁺ = 420.2 m/z

### A3 (illustrated for example 12)

A microwave vial was heat gun-dried and charged with bis(trichloromethyl) carbonate (26.6 mg, 89.6 µmol) and sodium bicarbonate (32.3 mg, 384 µmol). The flask was placed under argon and DCM (1 ml) was added to give a suspension. The suspension was cooled by an ice-bath and (1R,5S,6r)-6-((2-chloro-4-fluorophenoxy)methyl)-3-azabicyclo[3.1.0]hexane hydrochloride **(BB13,** 35.6 mg, 128 µmol) was added to the reaction. The mixture was stirred at 0 °C for 15 min and at room temperature overnight. The suspension turned into a clear solution overnight. The reaction mixture was then cooled down in an-ice bath and DCM (500 µl) and DIPEA (49.7 mg, 67.1 µl, 384 µmol) followed by (4aR,8aS)-hexahydro-2H-pyrido[4,3-b][1,4]oxazin-3(4H)-one (**BB1**, 20 mg, 128 µmol) were added. The resulting off-white suspension was stirred at room temperature for 6 hours.

The reaction mixture was poured on water and DCM and the layers were separated. The aqueous layer was extracted twice with DCM. The organic layers were dried over MgSO₄, filtered and evaporated to afford an orange oil (60 mg). The crude was purified by reverse-phase HPLC (method B7) to afford 24 mg (44.2%) of (4aR,8aS)-6-[(1R,5S,6r)-6-[(2-chloro-4-fluoro-phenoxy)methyl]-3-azabicyclo[3.1.0]hexane-3-carbonyl]-4,4a,5,7,8,8a-hexahydropyrido[4,3-b][1,4]oxazin-3-one as a white solid after lyophilisation. LC-MS (ESI): [M+H⁺]⁺ = 424.2 m/z
**HPLC Method B1**
   YMC-Triart C18, 12 nm, 5 µm, 100 x 30 mm, 15 min run time, gradient 15-35-50-100% ACN in Water + 0.1% HCOOH
**HPLC Method B2**
   YMC-Triart C18, 12 nm, 5 µm, 100 x 30 mm, 15 min run time, gradient 30-50-65-100% ACN in Water + 0.1% HCOOH
**HPLC Method B3**
   Gemini NX, 12 nm, 5 µm, 100 x 30 mm, 15 min gradient, ACN / Water + 0.1% TEA
**HPLC Method B4**
   YMC-Triart C18, 12 nm, 5 µm, 100 x 30 mm, 11 min run time, gradient 15-35-50-100% ACN in Water + 0.1% TEA
**HPLC Method B5**
   YMC-Triart C18, 12 nm, 5 µm, 100 x 30 mm, 9 min run time, gradient 40-60-80-100% ACN in Water + 0.1% HCOOH
**HPLC Method B6**
   YMC-Triart C18, 12 nm, 5 µm, 100 x 30 mm, 11 min run time, gradient 20-40-55-100% ACN in Water + 0.1% HCOOH
**HPLC Method B7**
   Gemini NX, 12 nm, 5 µm, 100 x 30 mm, 15 min gradient, ACN / Water + 0.1% HCOOH
**HPLC Method B8 (chiral)**
   Chiralpak AD, Normal Phase (isocratic): 65% Heptane; 35% Ethanol + NH4Oac
**HPLC Method B9**
   Gemini NX, 12 nm, 5 µm, 100 x 30 mm, 15 min run time, gradient 30-50-65-100% ACN in Water + 0.1% HCOOH
**Chiral SFC Method C1**
   OD-H, 12 nm, 5 µm, 250 x 4.6 mm, 30% iPrOH
Chiral SFC Method C2
   AD-H, 12 nm, 5 µm, 250 x 4.6 mm, 40% MeOH
Chiral SFC Method C3
   OZ-H, 12 nm, 5 µm, 250 x 4.6 mm, 35% iPrOH + 0.2% DEA
Chiral SFC Method C4
   IB, 12 nm, 5 µm, 250 x 4.6 mm, 9% MeOH, 100 ml/min
Chiral SFC Method C5
   IC, 12 nm, 5 µm, 250 x 4.6 mm, 30% MeOH

| **Ex.** | **Building Blocks** | **Synthesis and Purification Method** | **ESI(MS)** |
|---|---|---|---|
| **1** | BB2a | A2 | m/z = 420.2[ M+H⁺]⁺ |
| | CAS: 1889054-91-4 | B1 | |
| **2** | BB2a | A2 | m/z = 400.3 [M+H⁺]⁺ |
| | CAS: 1893326-25-4 | B2 | |
| **3** | BB1a | A1 | m/z = 435.2 [M+H⁺]⁺ |
| | CAS: 1422449-96-4 | B3 | |
| **4** | BB1a | A1 | m/z = 503.3 [M+H⁺]⁺ |
| | CAS: 1825117-24-5 | B4 | |
| **5** | BB1a | A1 | m/z = 487.1 [M+H⁺]⁺ |
| | CAS: 1822096-52-5 | B5 | |
| **6** | BB1a | A1 | m/z = 426.3 [M+H⁺]⁺ |
| | CAS: 2075635-92-4 | B1 | |
| **7** | BB1a | A1 | m/z = 494.3 [M+H⁺]⁺ |
| | BB9 | B2 | |
| **8** | BB1a | A1 | m/z = 460.2 [M+H⁺]⁺ |
| | BB10 | B6 | |
| **9** | BB2a | A2 | m/z = 441.3 [M+H⁺]⁺ |
| | BB11 | C1 | |
| | | Product is the first eluting of the two diastereomers. | |
| **10** | BB2a | A2 | m/z = 461.3 [M+H⁺]⁺ |
| | BB12 | C2 | |
| | | Product is the first eluting of the two diastereomers. | |
| **11** | BB2a | A2 | m/z = 461.3 [M+H⁺]⁺ |
| | BB12 | C2 | |
| | | Product is the second eluting of the two diastereomers. | |
| **12** | BB1a | A3 | m/z = 424.2 [M+H⁺]⁺ |
| | BB13 | B7 | |
| **13** | BB2a | A2 | m/z = 452.2 [M+H⁺]⁺ |
| | BB14 | B7 | |
| **14** | BB2a | A2 | m/z = 438.3 [M+H⁺]⁺ |
| | BB15 | B3 | |
| **15** | BB1a | A1 | m/z = 452.18 [M+H⁺]⁺ |
| | BB16 | flash chromatography (SiO₂, 0% to 10% MeOH in DCM) | |
| **16** | BB1a | A1 | m/z = 486.20 [M+H⁺]⁺ |
| | BB17 | flash chromatography (SiO₂, 0% to 10% MeOH in DCM) | |
| **17** | BB1a | A1 | m/z = 472.19 [M+H⁺]⁺ |
| | BB18 | flash chromatography (SiO₂, 0% to 10% MeOH in DCM) | |
| **18** | BB1a | A1 | m/z = 472.3 [M+H⁺]⁺ |
| | BB19 | B8 | |
| | | Product is the second eluting of the two diastereomers. | |
| **19** | BB1a | A1 | m/z = 472.3 [M+H⁺]⁺ |
| | BB20 | B8 | |
| | | Product is the second eluting of the two diastereomers. | |
| **20** | BB1a | A1 | m/z = 472.4 [M+H⁺]⁺ |
| | BB21 | C3 | |
| | | Product is the first eluting of the two diastereomers. | |
| **21** | BB1a | A1 | m/z = 472.4 [M+H⁺]⁺ |
| | BB21 | C3 | |
| | | Product is the second eluting of the two diastereomers. | |
| **22** | BB1a | A1 | m/z = 472.2 [M+H⁺]⁺ |
| | BB22 | flash chromatography (SiO₂, 0% to 10% MeOH in DCM) | |
| **23** | BB1a | A1 | m/z = 502.20 [M+H⁺]⁺ |
| | BB23 | flash chromatography (SiO₂, 0% to 10% MeOH in DCM) | |
| **24** | BB1a | A1 | m/z = 486.41 [M+H⁺]⁺ |
| | BB24 | flash chromatography (SiO₂, 0% to 10% MeOH in DCM) | |
| **25** | BB1a | A1 | m/z = 458.17 [M+H⁺]⁺ |
| | BB25 | flash chromatography (SiO₂, 0% to 10% MeOH in DCM) | |
| **26** | BB2a | A2 | m/z = 452.2 [M+H⁺]⁺ |
| | BB26 | B9 | |
| **27** | BB2b | A2 | m/z = 452.2 [M+H⁺]⁺ |
| | BB14 | B7 | |
| **28** | BB2a | A2 | m/z = 441.3 [M+H⁺]⁺ |
| | BB11 | C1 | |
| | | Product is the second eluting of the two diastereomers. | |

### Synthesis of building blocks

### BB1a & BB1b

### (+)-cis-4a,5,6,7,8,8a-Hexahydro-4H-pyrido[4,3-b][1,4]oxazin-3-one and (-)-cis-4a,5,6,7,8,8a-Hexahydro-4H-pyrido[4,3-b][1,4]oxazin-3-one

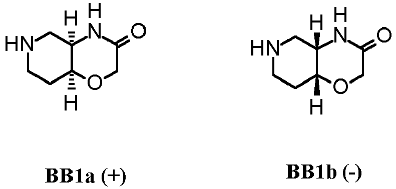

The enantiomers of rac-(4aR,8aS)-hexahydro-2H-pyrido[4,3-b][1,4]oxazin-3(4H)-one dihydrochloride (BB1, CAS 1909295-00-6, 500 mg, 2.18 mmol) were separated by preparative chiral HPLC (ReprosilChiral NR column) using an isocratic mixture of EtOH (containing 0.05% of NHaOAc) : n-heptane (30 : 70).

First eluting enantiomer: (+)-cis-4a,5,6,7,8,8a-Hexahydro-4H-pyrido[4,3-b][1,4]oxazin-3-one (BB1a). Yellow solid (0.150 g; 44.0%). MS (ESI): m/z = 157.1 [M+H]⁺.

Second eluting enantiomer: (-)-cis-4a,5,6,7,8,8a-Hexahydro-4H-pyrido[4,3-b][1,4]oxazin-3-one. (BB1b). Yellow solid (0.152 g; 44.6%). MS (ESI): m/z = 157.1 [M+H]⁺.

### BB2a & BB2b

### (4-nitrophenyl) (4aR,8aS)-3-oxo-4,4a,5,7,8,8a-hexahydropyrido[4,3-b][1,4]oxazine-6-carboxylate and (4-nitrophenyl) (4aS,8aR)-3-oxo-4,4a,5,7,8,8a-hexahydropyrido[4,3-b][1,4]oxazine-6-carboxylate

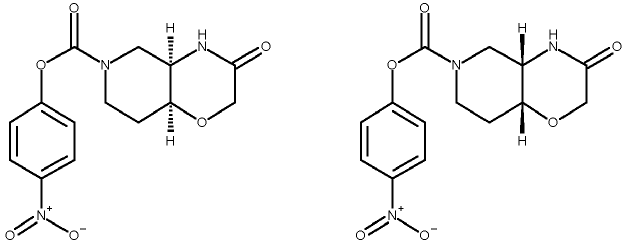

A suspension of rac-(4aR,8aS)-hexahydro-2H-pyrido[4,3-b][1,4]oxazin-3(4H)-one dihydrochloride (BB1, 4.5 g, 19.6 mmol) in dry DCM (125 ml) was cooled down to 0°C and DIPEA (6.35 g, 8.58 ml, 49.1 mmol) was added, followed by 4-nitrophenyl carbonochloridate (4.35 g, 21.6 mmol). The reaction mixture was stirred at 0°C for 10 min and for 2 hours at room temperature. The crude reaction was diluted with dichloromethane, transfered into a separating funnel for extraction with sat. aq. Na₂CO₃. The organic phase was collected and the aqueous phase was back-extracted with dichloromethane. The combined organic phases were dried over sodium sulfate and evaporated down to dryness to yield 6.62 g of a crude racemic product as a yellow solid. The crude material was directly submitted for a chiral SFC separation (Method C4) to yield the two enantiomers:
First eluting enantiomer, light beige solid (3.25g), contaminated with the second enantiomer.

The fraction was submitted for another SFC chiral separation (Method C5) to yield 2.71 g of the desired enantiomer (**BB2a**).

### Second eluting enantiomer, yellow solid (BB2b, 2.72 g)

### BB9

### (1R,5S,6r)-6-[1-[4-fluoro-3-(trifluoromethyl)phenyl]pyrazol-3-yl]-3-azabicyclo[3.1.0]hexane 2,2,2-trifluoroacetate

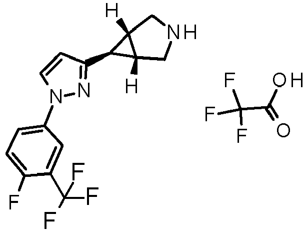

In a 20 ml tube under argon, tert-butyl (1R,5S,6r)-6-(1-(4-fluorophenyl)-1H-pyrazol-3-yl)-3-azabicyclo[3.1.0]hexane-3-carboxylate (120mg, 349 µmol) was dissolved in DCM (2 ml), TFA (239 mg, 162 µl, 2.1 mmol) was added and the reaction stirred at room temperature for 16hr, and then concentrated *in vacuo* (azeotrop with toluene) to yield the desired product (122 mg, 98%). Used directly in next step. LC-MS (ESI): m/z = 244.2 [M+H⁺]⁺

### a) tert-butyl (1R,5S,6r)-6-((E)-3-(dimethylamino)acryloyl)-3-azabicyclo[3.1.0]hexane-3-carboxylate

tert-butyl (1R,5S,6r)-6-acetyl-3-azabicyclo[3.1.0]hexane-3-carboxylate (CAS 2075712-96-6, 990 mg, 4.39 mmol) was combined with N,N-dimethylformamide dimethyl acetal (3.24 g, 3.64 ml, 26.4 mmol) and heated at 100°C over night for 15 hrs. The mixture was cooled to room temperature and directly evaporated *in vacuo.* The residue was extracted with ethyl acetate / water, brine, organic fractions were combined and dried over MgSO₄, filtered and concentrated under reduced pressure to afford the deisred product (1.21 g, 98%) as a yellow oil, LC-MS (ESI): m/z = 281.3 [M+H⁺]⁺

### b) tert-butyl (1R,5S,6r)-6-(1H-pyrazol-3-yl)-3-azabicyclo[3.1.0]hexane-3-carboxylate

In a 20ml tube, tert-butyl (1R,5S,6r)-6-((E)-3-(dimethylamino)acryloyl)-3-azabicyclo[3.1.0]hexane-3-carboxylate (1.21 g, 4.32 mmol) was dissolved in ethanol (6 ml) and hydrazine hydrate (302 mg, 293 µl, 6.04 mmol) was added. The reaction was stirred 1 hr at 80°C. Solvent was removed under reduced pressure, purification by preparative SFC (Princeton 2-Ethyl Pyridine column, 30x250 mm, 5% EtOH + 0.2% DEA) yielded the desired product as a yellow, viscous oil (890 mg, 83%). LC-MS (ESI): m/z = 250.1 [M+H⁺]⁺

### c) tert-butyl (1R,5S,6r)-6-(1-(4fluorophenyl)-1H-pyrazol-3-yl)-3-azabicyclo[3.1.0]hexane-3-carboxylate

In a 100 ml flask under argon, tert-butyl (1R,5S,6r)-6-(1H-pyrazol-3-yl)-3-azabicyclo[3.1.0]hexane-3-carboxylate (300mg, 1.2 mmol) was suspended in DMF (8 ml), pyridine (381 mg, 389 µl, 4.81 mmol), (4-fluorophenyl)boronic acid (219 mg, 1.56 mmol) and copper (II) acetate (328 mg, 1.8 mmol) were added. The green solution was stirred 60 hr at room temperature. Solvent was removed *in vacuo,* the crude was extracted with ethyl acetat / water / sat. NaCl, organic fractions were dried over MgSO₄, filtered and solvent was removed. Purification by flash chromatography (SiO₂, Heptane / ethyl acetate 0 to 40% in 40min) led to the desired product as a colorless, solid (317 mg, 77%). LC-MS (ESI): m/z = 288.1 [M-56+H⁺]⁺

### BB10

### (1R,5S,6r)-6-[1-[2-chloro-4-fluoro-phenyl]pyrazol-3-yl]-3-azabicyclo[3.1.0]hexane 2,2,2-trifluoroacetate

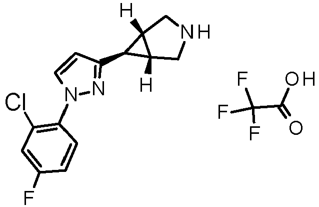

BB10 was prepared similarly as described for BB9, using tert-butyl (1R,5S,6r)-6-(1H-pyrazol-3-yl)-3-azabicyclo[3.1.0]hexane-3-carboxylate and (2-chloro-4-fluorophenyl)boronic acid in step c).

LC-MS (ESI) for BB10: m/z = 278.2 [M+H⁺]⁺

### BB11

### rac-3-((((1R,4R,5S)-2-azabicyclo[2.2.1]heptan-5-yl)oxy)methyl)-5,6,7,8-tetrahydroisoquinoline bis(2,2,2-trifluoroacetate)

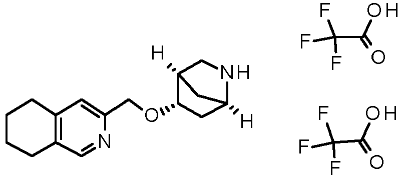

In a 20 ml tube under argon, rac-tert-butyl (1R,4R,5S)-5-((5,6,7,8-tetrahydroisoquinolin-3-yl)methoxy)-2-azabicyclo[2.2.1]heptane-2-carboxylate (40mg, 112 µmol) was dissolved in DCM (1 ml), TFA (69 µl, 0.89 mmol) was added and the reaction stirred at room temperature for 24 hr, and then concentrated *in vacuo* (azeotrop with toluene) to yield the desired product (60 mg, 90% pure, 99.5%) as a light yellow oil. Used directly in next step. LC-MS (ESI): m/z = 259.3 [M+H⁺]⁺

### a) 3-(bromomethyl)-5,6,7,8-tetrahydroisoquinoline

Appel reaction: To a solution of (5,6,7,8-tetrahydroisoquinolin-3-yl)methanol (169 mg, 1.04 mmol) in dry DCM (2.91 ml) was added CBr₄ (412 mg, 1.24 mmol), the mixture was cooled to 0-3°C and within 10 min triphenylphosphine (353 mg, 1.35 mmol) in 1ml dry DCM was added. Stirring continued for 1 hr at 2-4°C, then 20ml DCM and silica gel were added, the solvent was removed *in vacuo* and the crude was purified by flash chromatography (SiO₂, heptane/ethylacetate 0 to 50% in 40min). The desired product was obtained as 190 mg (81%) of a light red solid. LC-MS (ESI): m/z = 226.1 [M+H⁺]⁺

### b) rac-tert-butyl (1R,4R,5S)-5-((5,6,7,8-tetrahydroisoquinolin-3-yl)methoxy)-2-azabicyclo[2.2.1]heptane-2-carboxylate

To a solution of rac-tert-butyl (1S,4S,5R)-5-hydroxy-2-azabicyclo[2.2.1]heptane-2-carboxylate (CAS 198835-03-9, 197 mg, 924 µmol) in dry THF (2.79 ml) was added potassium tert-butoxide 1M in THF (924 µl 924 µmol) and the turbid reaction mixture was stirred at r.t. for 30 min followed by addition of 3-(bromomethyl)-5,6,7,8-tetrahydroisoquinoline (190 mg, 840 µmol) at 0-2°C. The reaction mixture was 3 hr stirred at 3-6°C, then diluted with ethyl acetate and extracted with water, the organic phase was collected and the aqueous phase was back-extracted with ethyl acetate. The combined organic phases were dried over sodium sulfate and evaporated down to dryness. The crude material was purified by prep. HPLC (Gemini NX, 12 nm, 5 µm, 100 x 30 mm, ACN / Water+0.1% TEA) to get the desired product (40 mg, 13.3%) as a light brown solid. LC-MS (ESI): m/z = 359.3 [M+H⁺]⁺

### BB12

### rac-(1R,4R,5S)-5-[(5-chloro-4-cyclopropyl-2-pyridyl)methoxy]-2-azabicyclo[2.2.1]heptane bis(2,2,2-trifluoroacetate)

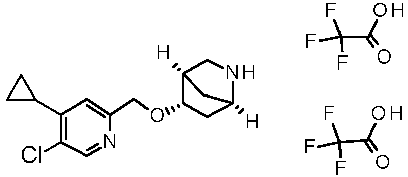

In a 20 ml tube under argon, rac-tert-butyl (1R,4R,5S)-5-((5-chloro-4-cyclopropylpyridin-2-yl)methoxy)-2-azabicyclo[2.2.1]heptane-2-carboxylate (272mg, 718 µmol) was dissolved in DCM (2 ml), TFA (332 µl, 0.4.3 mmol) was added and the reaction stirred at room temperature for 24 hr, and then concentrated *in vacuo* (azeotrop with toluene) to yield the desired product (381 mg, 90% pure, 94%) as a light yellow oil. Used directly in next step. LC-MS (ESI): m/z = 279.2 [M+H⁺]⁺

### a) 2-(bromomethyl)-5-chloro-4-cyclopropylpyridine

Appel reaction: To a solution of (5-chloro-4-cyclopropylpyridin-2-yl)methanol (290 mg, 1.58 mmol) in dry DCM (5 ml) was added CBr₄ (628 mg, 1.9 mmol), the mixture was cooled to 0-3°C and within 10 min triphenylphosphine (538 mg, 2.05 mmol) in 1ml dry DCM was added. Stirring continued for 1 hr at 2-4°C, then 20ml DCM and silica gel were added, the solvent was removed *in vacuo* and the crude was purified by flash chromatography (SiO₂, heptane/ethylacetate 0 to 40% in 40min). The desired product was obtained as 345 mg (90% pure, 80%) of a light red semisolid. LC-MS (ESI): m/z = 248.0 [M+H⁺]⁺

### b) rac-tert-butyl (1R,4R,5S)-5-((5-chloro-4-cyclopropylpyridin-2-yl)methoxy)-2-azabicyclo[2.2.1]heptane-2-carboxylate

To a solution of tert-butyl 5-hydroxy-2-azabicyclo[2.2.1]heptane-2-carboxylate (CAS 198835-03-9, 324 mg, 1.52 mmol) in dry THF (5 ml) was added potassium tert-butoxide 1M in THF (1.52 ml, 1.52 mmol) and the turbid reaction mixture was stirred at r.t. for 30 min followed by addition of 2-(bromomethyl)-5-chloro-4-cyclopropylpyridine (340 mg, 1.38 mmol) at 0-2°C. The reaction mixture was 3 hr stirred at 3-6°C, then diluted with ethyl acetate and extracted with water, the organic phase was collected and the aqueous phase was back-extracted with ethyl acetate. The combined organic phases were dried over sodium sulfate and evaporated down to dryness. The crude material was purified by flash chromatography (silica gel, 0% to 50% in 40min EtOAc in heptane) to get the desired product (272 mg, 95% pure, 49.4%) as a light brown oil. LC-MS (ESI): m/z = 379.2 [M+H⁺]⁺

### BB13

### (1R,5S,6r)-6-[(2-chloro-4-fluoro-phenoxy)methyl]-3-azabicyclo[3.1.0]hexane;hydrochloride

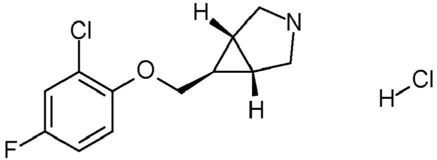

To a solution of tert-butyl (1R,5S,6r)-6-[(2-chloro-4-fluoro-phenoxy)methyl]-3-azabicyclo[3.1.0]hexane-3-carboxylate (150.0 mg, 0.440 mmol) in EtOAc (2 mL) was added 9M HCl in EtOAc (0.4 mL, 3.6 mmol) at 0 °C. The solution was stirred at 15°C for 2.5 hrs. The solution was concentrated, then dried by lyophilization *in vacuo* to obtained desired product (1R,5S,6r)-6-[(2-chloro-4-fluoro-phenoxy)methyl]-3-azabicyclo[3.1.0]hexane hydrochloride (56.2 mg, 0.200 mmol, 44.94% yield as a white solid. LC-MS (ESI): m/z = 242.0 [M+H⁺]⁺

### a) tert-butyl (1R,5S,6r)-6-[(2-chloro-4-fluoro-phenoxy)methyl]-3-azabicyclo[3.1.0]hexane-3-carboxylate

To a solution of tert-butyl (1R,5S,6r)-6-(methylsulfonyloxymethyl)-3-azabicyclo[3.1.0]hexane-3-carboxylate (CAS 958633-11-9, 294 mg, 1.01 mmol) in DMF (3 mL) was added cesium carbonate (493.16 mg, 1.51 mmol), 2-chloro-4-fluorophenol (0.11 mL, 1.11 mmol) at 15 °C. The mixture was heated to 85 °C and stirred for 16 hrs. The mixture was quenched with water (5 mL) at 0 °C, then extracted three times with EtOAc (5 mL) and dried over Na₂SO₄. The organic layers were combined and concentrated *in vacuo* to obtained crude product as a yellow oil. The crude product was purified by Prep-HPLC and dried by lyophilization to obtain the desired product tert-butyl (1R,5S,6r)-6-[(2-chloro-4-fluoro-phenoxy)methyl]-3-azabicyclo[3.1.0]hexane-3-carboxylate (150 mg, 0.440 mmol, 43.4% yield) as a light yellow oil. LC-MS (ESI): m/z = 286.0 [M-56+H⁺]⁺

### BB14

### 8-[(2-chloro-4-fluoro-phenoxy)methyl]-3-azabicyclo[3.2.1]octane 2,2,2-trifluoroacetate

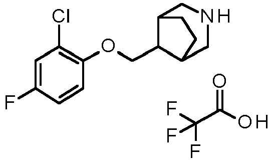

Boc-deprotection was performed as described e.g. for **BB12.** Crude product was obtained as a light yellow oil and was used directly for next step (Yield 99%, Purity 80%). LC-MS (ESI): m/z = 270.2 [M+H⁺]⁺

### a) tert-butyl 8-((2-chloro-4-fluorophenoxy)methyl)-3-azabicyclo[3.2.1]octane-3-carboxylate

To a solution of 2-chloro-4-fluorophenol (65.6 mg, 48.8 µl, 448 µmol), tert-butyl 8-(hydroxymethyl)-3-azabicyclo[3.2.1]octane-3-carboxylate (108 mg, 448 µmol) and triphenylphosphine (117 mg, 448 µmol) in DCM (2.24 ml) was added DIAD (99.5 mg, 95.7 µl, 492 µmol) dropwise and the reaction was stirred for 19 h at rt. The reaction mixture was quenched by addition of sat. aq. NaHCO₃ solution. The phases were separated and the aq. phase was extracted with DCM three times. The combined organic layers were dried over sodium sulfate and concentrated to dryness to afford a brown oil. The crude was immobilized on Isolute and purified by column chromatography (SiO₂, 0 - 30 % EtOAc in heptane) to afford tert-butyl 8-((2-chloro-4-fluorophenoxy)methyl)-3-azabicyclo[3.2.1]octane-3-carboxylate (45.9 mg, 26.3 %) as a yellow oil. LC-MS (ESI): m/z = 314.2 [M-56+H⁺]⁺

### BB15

### 5-(2-chloro-4-fluorophenoxy)octahydrocyclopenta[c]pyrrole 2,2,2-trifluoroacetate

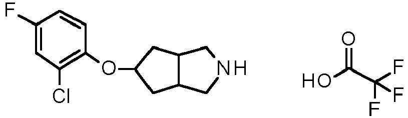

Synthesis was done as described for **BB14,** starting from 2-chloro-4-fluorophenol (64.5 mg, 440 umol) and tert-butyl 5-hydroxyhexahydrocyclopenta[c]pyrrole-2(1H)-carboxylate (100 mg, 440 umol. Product was obtained as a light-brown oil, yield after Boc-deprotection was 149.8 mg (purity 82%). LC-MS (ESI): m/z = 256.2 [M+H⁺]⁺

### BB16

### 5-((2-chloro-4-fluorobenzyl)oxy)octahydrocyclopenta[c]pyrrole 2,2,2-trifluoroacetate

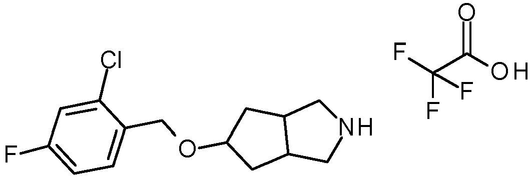

**BB16** was prepared similarly as described for **BB11** (step b and Boc-deprotection), using tert-butyl 5-hydroxyhexahydrocyclopenta[c]pyrrole-2(1H)-carboxylate and 1-(bromomethyl)-2-chloro-4-fluorobenzene.

LC-MS (ESI) for BB16: m/z = 270.2 [M+H⁺]⁺

### BB17

### 5-((2-fluoro-4-(trifluoromethyl)benzyl)oxy)octahydrocyclopenta[c]pyrrole 2,2,2-trifluoroacetate

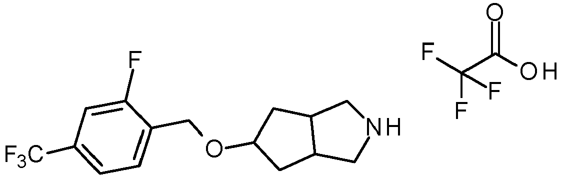

**BB17** was prepared similarly as described for **BB11** (step b and Boc-deprotection), using tert-butyl 5-hydroxyhexahydrocyclopenta[c]pyrrole-2(1H)-carboxylate and 1-(bromomethyl)-2-fluoro-4-(trifluoromethyl)benzene.

LC-MS (ESI) for BB17: m/z = 304.2 [M+H⁺]⁺

### BB18

### 5-(2-fluoro-4-(trifluoromethyl)phenoxy)octahydrocyclopenta[c]pyrrole 2,2,2-trifluoroacetate

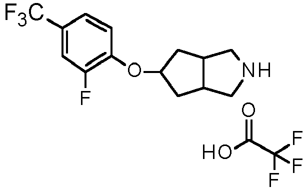

In a 20 ml tube under argon, tert-butyl 5-(2-fluoro-4-(trifluoromethyl)phenoxy)hexahydrocyclopenta[c]pyrrole-2(1H)-carboxylate (0.290 g, 745 µmol) was dissolved in DCM (4 ml), TFA (574 µl, 7.45 mmol) was added and the reaction stirred at room temperature for 1 hr, and then concentrated *in vacuo* (azeotrop with toluene) to yield the desired product (321 mg, 90% pure, 96%) as a colorless oil. Used directly in next step. LC-MS (ESI): m/z = 290.2 [M+H⁺]⁺

### a) tert-butyl 5-(2-fluoro-4-(trifluoromethyl)phenoxy)hexahydrocyclopenta[c]pyrrole-2(1H)-carboxylate

In a 25m1 four-necked flask under argon, tert-butyl 5-hydroxyhexahydrocyclopenta[c]pyrrole-2(1H)-carboxylate (0.250 g, 1.1 mmol) was dissolved in THF (5 ml), and 2-fluoro-4-(trifluoromethyl)phenol (198 mg, 138 µl, 1.1 mmol) and triphenylphosphine (317 mg, 1.21 mmol) were added. The clear solution was stirred 5 min at RT, then cooled to 0-2°C and slowly DEAD (211 mg, 192 µl, 1.21 mmol) was added within 10min, stirring continued for 1 hr at 2-4°C and overnight at RT. 50ml diethylether were added to the reaction mixture, which was extracted with 2x25ml water, 2x 20ml sat NaOH and 1x 20ml brine. The organic fraction was dried over Mg₂SO₄, solvent was removed *in vacuo.* The crude material was purified by flash chromatography (silica gel, 0% to 30% EtOAc in heptane), yielding the product as a colorless oil (290 mg, 67.7%). LC-MS (ESI): m/z = 334.2 [M-56+H⁺]⁺

### BB19

### Rac-(1S,4R,6R)-6-((2-fluoro-4-(trifluoromethyl)benzyl)oxy)-2-azabicyclo[2.2.1]heptane 2,2,2-trifluoroacetate

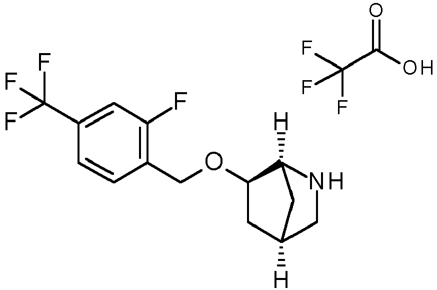

Boc-deprotection was performed as described e.g. for **BB18.** Crude product was obtained as a light yellow oil and was used directly for next step (Yield 87%). LC-MS (ESI): m/z = 290.3 [M+H⁺]⁺

### a) rac-tert-butyl (1R,4S,6S)-6-((2-fluoro-4-(trifluoromethyl)benzyl)oxy)-2-azabicyclo[2.2.1]heptane-2-carboxylate

To a solution of rac-tert-butyl (1R,4S,6S)-6-hydroxy-2-azabicyclo[2.2.1]heptane-2-carboxylate (Synthonix, CAS 198835-05-1; 0.300 g, 1.41 mmol) in THF (5 ml) was added potassium tert-butoxide 1M solution in THF (1.48 ml, 1.48 mmol) and the turbid reaction mixture was stirred at r.t for 15min followed by addition of 1-(bromomethyl)-2-fluoro-4-(trifluoromethyl)benzene (362 mg, 1.41 mmol). The reaction mixture was then stirred at r.t for 60 hr. The crude reaction was diluted with ethyl acetate and extracted with water, the organic phase was collected and the aqueous phase was back-extracted with ethyl acetate. The combined organic phases were dried over sodium sulfate and evaporated down to dryness. The crude material was purified by flash chromatography (silica gel, 0% to 80% EtOAc in heptane), yielding the desired product as a colorless oil (237 mg, 43%). LC-MS (ESI): m/z = 334.2 [M-56+H⁺]⁺

### BB20

### Rac-(1S,4R,6S)-6-((2-fluoro-4-(trifluoromethyl)benzyl)oxy)-2-azabicyclo[2.2.1]heptane 2,2,2-trifluoroacetate

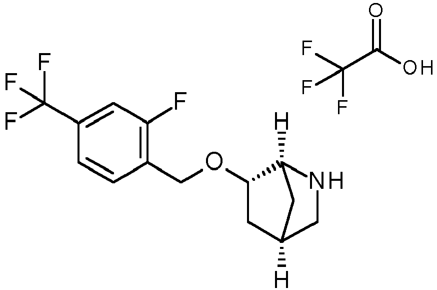

**BB20** was prepared similarly as described for **BB19,** using rac-tert-butyl (1R,4S,6R)-6-hydroxy-2-azabicyclo[2.2.1]heptane-2-carboxylate (Combi-Blocks QE-8592, CAS : 207405-59-2) and 1-(bromomethyl)-2-fluoro-4-(trifluoromethyl)benzene.

LC-MS (ESI) for BB20: m/z = 290.3 [M+H⁺]⁺

### BB21

### Rac-(1R,4R,5S)-5-((2-fluoro-4-(trifluoromethyl)benzyl)oxy)-2-azablcyclo[2.2.1]heptane 2,2,2-trifluoroacetate

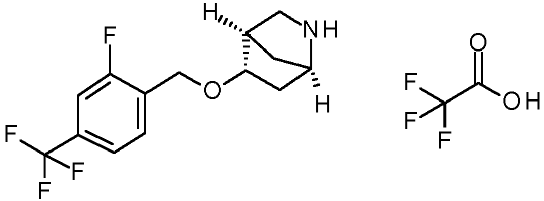

**BB21** was prepared similarly as described for **BB19,** using rac-tert-butyl (1R,4R,5S)-5-hydroxy-2-azabicyclo[2.2.1]heptane-2-carboxylate (AbovChem, CAS : 198835-03-9) and 1-(bromomethyl)-2-fluoro-4-(trifluoromethyl)benzene.

LC-MS (ESI) for BB21: m/z = 290.3 [M+H⁺]⁺

### BB22

### 6-((2-fluoro-4-(trifluoromethyl)benzyl)oxy)-3-azabicyclo[3.1. 1]heptane 2,2,2-trifluoroacetate

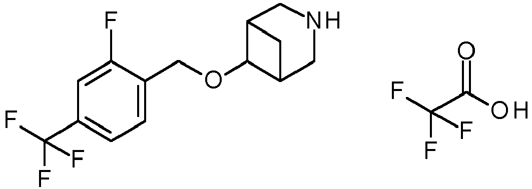

**BB22** was prepared similarly as described for **BB19,** using tert-butyl 6-hydroxy-3-azabicyclo[3.1.1]heptane-3-carboxylate (Spirochem, CAS: 1357353-36-6) and 1-(bromomethyl)-2-fluoro-4-(trifluoromethyl)benzene.

LC-MS (ESI) for BB21: m/z = 290.2 [M+H⁺]⁺

### BB23

### (1R,5S,7s)-7-((2-fluoro-4-(trifluoromethyl)benzyl)oxy)-3-oxa-9-azabicyclo[3.3.1]nonane 2,2,2-trifluoroacetate

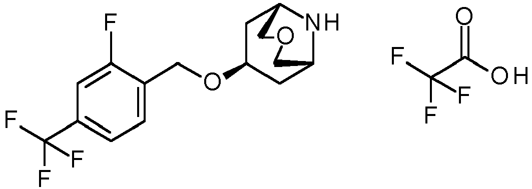

**BB23** was prepared similarly as described for **BB19,** using tert-butyl (1R,5S,7s)-7-hydroxy-3-oxa-9-azabicyclo[3.3.1]nonane-9-carboxylate (endo, Spirochem, CAS: 1148006-31-8) and 1-(bromomethyl)-2-fluoro-4-(trifluoromethyl)benzene.

LC-MS (ESI) for BB21: m/z = 320.2 [M+H⁺]⁺

### BB24

### (1R,3R,SS)-3-((2-fluoro-4-(trifluoromethyl)benzyl)oxy)-8-azabicyclo[3.2.1]octane 2,2,2-trifluoroacetate

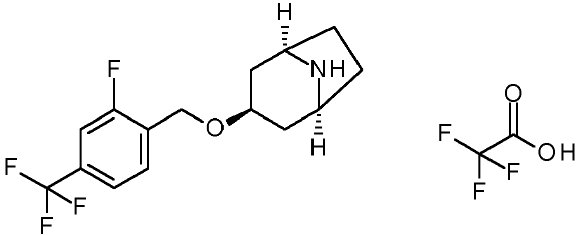

**BB24** was prepared similarly as described for **BB19,** using tert-butyl (1R,3r,5S)-3-hydroxy-8-azabicyclo[3.2.1]octane-8-carboxylate (endo, Spirochem, CAS: 143557-91-9) and 1-(bromomethyl)-2-fluoro-4-(trifluoromethyl)benzene.

LC-MS (ESI) for BB21: m/z = 304.2 [M+H⁺]⁺

### BB25

### (1R,5S,6r)-6-((2-fluoro-4-(trifluoromethyl)phenoxy)methyl)-3-azabicyclo[3.1.0]hexane 2,2,2-trifluoroacetate

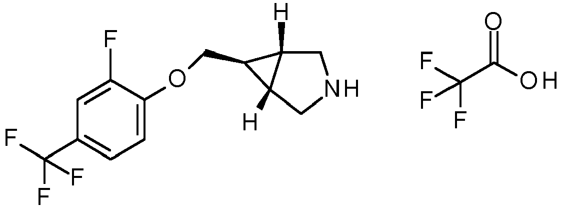

**BB25** was prepared similarly as described for **BB18,** using tert-butyl (1R,SS,6r)-6-(hydroxymethyl)-3-azabicyclo[3.1.0]hexane-3-carboxylate and 2-chloro-4-fluorophenol

LC-MS (ESI) for BB25: m/z = 276.2 [M+H⁺]⁺

### BB26

### 3-((2-chloro-4-fluorophenoxy)methyl)-8-azabicyclo[3.2.1]octane hydrochloride

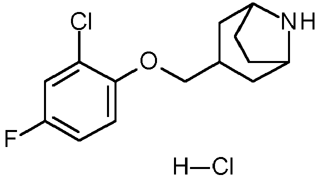

**BB26** was prepared similarly as described for **BB13,** using tert-butyl 8-(methylsulfonyloxymethyl)-3-azabicyclo[3.2.1]octane-3-carboxylate and 2-chloro-4-fluorophenol

LC-MS (ESI) for BB26: m/z = 270. 0 [M+H⁺]⁺

### Example 29

A compound of formula (I) can be used in a manner known per se as the active ingredient for the production of tablets of the following composition:

| | Per tablet |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example 30

A compound of formula (I) can be used in a manner known per se as the active ingredient for the production of capsules of the following composition:

| | Per capsule |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

## Claims

1. A compound of formula (I) 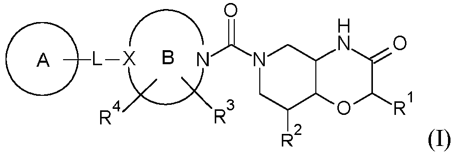 or a pharmaceutically acceptable salt thereof, wherein:
X is N or C-R⁵;
L is selected from a covalent bond, -(CH₂)ₙ-O-, -O-(CH₂)ₚ-, and -SO₂-;
n is an integer selected from 0, 1, 2 and 3;
p is an integer selected from 1, 2 and 3;
A is:
(i) C₆₋₁₄-aryl substituted with R⁶, R⁷ and R⁸; or
(ii) 5-14 membered heteroaryl substituted with R⁹, R¹⁰ and R¹¹; or
B is a bridged bicyclic heterocycle;
R¹ is hydrogen or C₁₋₆-alkyl;
R² is hydrogen or C₁₋₆-alkyl;
R³ is hydrogen, C₁₋₆-alkyl, halo-C₁₋₆-alkyl, halogen or hydroxy;
R⁴ is hydrogen, C₁₋₆-alkyl, halo-C₁₋₆-alkyl, halogen or hydroxy;
R⁵ is hydrogen, C₁₋₆-alkyl, halo-C₁₋₆-alkyl, halogen or hydroxy; and
R⁶, R⁷, R⁸, R⁹, R¹⁰, and R¹¹ are independently selected from hydrogen, C₁₋₆-alkyl, halo-C₁₋₆-alkyl, halogen, C₁₋₆-alkoxy, halo-C₁₋₆-alkoxy, SF₅, C₁₋₆-alkylsulfonyl, cyano, C₃₋₁₀-cycloalkyl, C₆₋₁₄-aryl, and 5-14 membered heteroaryl, wherein said C₃₋₁₀-cycloalkyl, C₆₋₁₄-aryl, and 5-14 membered heteroaryl are optionally substituted with 1-2 substituents selected from halogen, cyano, SF₅ C₁₋₆-alkyl, C₁₋₆-alkoxy, halo-C₁₋₆-alkyl, and halo-C₁₋₆-alkoxy.

2. The compound of formula (I) according to claim 1, or a pharmaceutically acceptable salt thereof, wherein R¹ and R² are both hydrogen.

3. The compound of formula (I) according to any one of claims 1 to 2, or a pharmaceutically acceptable salt thereof, wherein R³ and R⁴ are both hydrogen.

4. The compound of formula (I) according to any one of claims 1 to 3, or a pharmaceutically acceptable salt thereof, wherein
R⁹ is selected from hydrogen, C₃₋₁₀-cycloalkyl, and C₆₋₁₄-aryl, wherein said C₆₋₁₄-aryl is substituted with 1-2 substituents selected from halogen and halo-C₁₋₆-alkyl;
R¹⁰ is selected from hydrogen and halogen; and
R¹¹ is hydrogen.

5. The compound of formula (I) according to any one of claims 1 to 4, or a pharmaceutically acceptable salt thereof, wherein X is C-R⁵ and R⁵ is hydrogen.

6. The compound of formula (I) according to any one of claims 1 to 5, or a pharmaceutically acceptable salt thereof, wherein
L is selected from -(CH₂)ₙ-O- and -O-(CH₂)ₚ-;
n is an integer selected from 0 and 1; and
p is 1.

7. The compound of formula (I) according to any one of claims 1 to 6, or a pharmaceutically acceptable salt thereof, wherein
A is C₆₋₁₄-aryl substituted with R⁶, R⁷ and R⁸;
R⁶ is selected from halo-C₁₋₆-alkyl and halogen;
R⁷ is halogen; and
R⁸ is hydrogen.

8. The compound of formula (I) according to claim 7, or a pharmaceutically acceptable salt thereof, wherein
A is C₆₋₁₄-aryl substituted with R⁶, R⁷ and R⁸;
R⁶ is selected from hydrogen, C₁₋₆-alkyl, halo-C₁₋₆-alkyl, and halogen;
R⁷ is selected from hydrogen and halogen; and
R⁸ is hydrogen.

9. The compound of formula (I) according to claim 8, or a pharmaceutically acceptable salt thereof, wherein
A is phenyl substituted with R⁶, R⁷ and R⁸;
R⁶ is selected from CF₃, chloro, and fluoro;
R⁷ is fluoro or chloro; and
R⁸ is hydrogen.

10. The compound of formula (I) according to any one of claims 1 to 9, or a pharmaceutically acceptable salt thereof, wherein B is a 6-14 membered bridged bicyclic heterocycle comprising 1-3 heteroatoms selected from N, S, and O.

11. The compound of formula (I) according to claim 10, or a pharmaceutically acceptable salt thereof, wherein B is a 6-8 membered bridged bicyclic heterocycle comprising 1 nitrogen atom.

12. The compound of formula (I) according to claim 11, or a pharmaceutically acceptable salt thereof, wherein B is selected from 8-azabicyclo[3.2.1]octan-8-yl (**a**); 3,3a,4,5,6,6a-hexahydro-1H-cyclopenta[c]pyrrol-2-yl (**b**); 3-azabicyclo[3.1.0]hexan-3-yl (**c**); and 2-azabicyclo[2.2.1]heptan-2-yl (**d**): 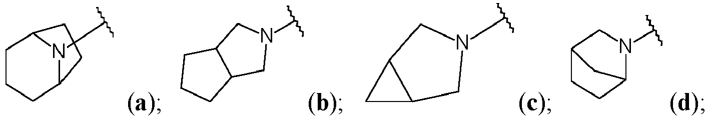 wherein a wavy line indicates the point of attachment to the carbonyl bridge bridging B to the hexahydropyrido oxazinone core of formula (I).

13. A compound selected from:
(4aR,8aS)-6-(5-(2-chlorophenoxy)-3,3a,4,5,6,6a-hexahydro-1H-cyclopenta[c]pyrrole-2-carbonyl)-4,4a,5,7,8,8a-hexahydropyrido[4,3-b] [1,4]oxazin-3-one;
(4aR,8aS)-6-(5-(2-methylphenoxy)-3,3a,4,5,6,6a-hexahydro-1H-cyclopenta[c]pyrrole-2-carbonyl)-4,4a,5,7,8,8a-hexahydropyrido[4,3-b] [1,4]oxazin-3-one;
(4aR,8aS)-6-(5-(benzenesulfonyl)-1,3,3a,4,6,6a-hexahydropyrrolo[3,4-c]pyrrole-2-carbonyl)-4,4a,5,7,8,8a-hexahydropyrido[4,3-b] [1,4]oxazin-3-one;
(4aR,8aS)-6-(5-[4-(trifluoromethyl)phenyl]sulfonyl-1,3,3a,4,6,6a-hexahydropyrrolo[3,4-c]pyrrole-2-carbonyl)-4,4a,5,7,8,8a-hexahydropyrido[4,3-b][1,4]oxazin-3-one;
(4aR, 8aS)-6-(5-(2-chloro-4-fluoro-phenyl)sulfonyl-1,3 ,3 a,4,6,6a-hexahydropyrrolo [3,4-c]pyrrole-2-carbonyl)-4,4a,5,7,8,8a-hexahydropyrido[4,3-b][1,4]oxazin-3-one;
(4aR,8aS)-6-[(1R,5S,6r)-6-[1-(4-fluorophenyl)pyrazol-3-yl]-3-azabicyclo[3.1.0]hexane-3-carbonyl]-4,4a,5,7,8,8a-hexahydropyrido[4,3-b] [1,4]oxazin-3-one;
(4aR,8aS)-6-[(1R,5S,6r)-6-[1-[4-fluoro-3-(trifluoromethyl)phenyl]pyrazol-3-yl]-3-azabicyclo[3.1.0]hexane-3-carbonyl]-4,4a,5,7,8,8a-hexahydropyrido[4,3-b][1,4]oxazin-3 -one;
(4aR,8aS)-6-[(1R,5S,6r)-6-[1-(2-chloro-4-fluoro-phenyl)pyrazol-3-yl]-3-azabicyclo[3.1.0]hexane-3-carbonyl]-4,4a,5,7,8,8a-hexahydropyrido[4,3-b][1,4]oxazin-3 -one;
(4aR,8aS)-6-[rel-(1R,4R, 5 S)-5 -(5, 6,7, 8-tetrahydroisoquinolin-3 -ylmethoxy)-2-azabicyclo[2.2.1]heptane-2-carbonyl]-4,4a,5,7,8,8a-hexahydropyrido[4,3-b][1,4]oxazin-3 -one;
(4aR,8aS)-6-[rel-(1R,4R,5S)-5-[(5-chloro-4-cyclopropyl-2-pyridyl)methoxy]-2-azabicyclo[2.2.1]heptane-2-carbonyl]-4,4a,5,7,8,8a-hexahydropyrido[4,3-b][1,4]oxazin-3-one;
(4aR,8aS)-6-[rel-(1R,4R,5S)-5-[(5-chloro-4-cyclopropyl-2-pyridyl)methoxy]-2-azabicyclo[2.2.1]heptane-2-carbonyl]-4,4a,5,7,8,8a-hexahydropyrido[4,3-b][1,4]oxazin-3 -one;
(4aR,8aS)-6-[(1R,5S,6r)-6-[(2-chloro-4-fluoro-phenoxy)methyl]-3-azabicyclo[3.1.0]hexane-3-carbonyl]-4,4a,5,7,8,8a-hexahydropyrido[4,3-b][1,4]oxazin-3 -one;
(4aR,8aS)-6-[8-[(2-chloro-4-fluoro-phenoxy)methyl]-3-azabicyclo[3.2.1]octane-3-carbonyl]-4,4a,5,7,8,8a-hexahydropyrido[4,3-b] [1,4]oxazin-3-one;
(4aR,8aS)-6-[5-(2-chloro-4-fluoro-phenoxy)-3,3a,4,5,6,6a-hexahydro-1H-cyclopenta[c]pyrrole-2-carbonyl]-4,4a,5,7,8,8a-hexahydropyrido[4,3-b][1,4]oxazin-3-one;
(4aR,8aS)-6-[5-[(2-chloro-4-fluoro-phenyl)methoxy]-3,3a,4,5,6,6a-hexahydro-1H-cyclopenta[c]pyrrole-2-carbonyl]-4,4a,5,7,8,8a-hexahydropyrido[4,3-b][1,4]oxazin-3-one;
(4aR, 8aS)-6-[5- [[2-fluoro-4-(trifluoromethyl)phenyl]methoxy] -3,3 a,4,5,6,6a-hexahydro-1H-cyclopenta[c]pyrrole-2-carbonyl]-4,4a,5,7,8,8a-hexahydropyrido[4,3-b][1,4]oxazin-3 -one;
(4aR,8aS)-6-[5-[2-fluoro-4-(trifluoromethyl)phenoxy]-3,3a,4,5,6,6a-hexahydro-1H-cyclopenta[c]pyrrole-2-carbonyl]-4,4a,5,7,8,8a-hexahydropyrido[4,3-b][1,4]oxazin-3-one;
(4aR,8aS)-6-[rel-(1R,4S,6S)-6-[[2-fluoro-4-(trifluoromethyl)phenyl]methoxy]-2-azabicyclo[2.2.1]heptane-2-carbonyl]-4,4a,5,7,8,8a-hexahydropyrido[4,3-b][1,4]oxazin-3-one;
(4aR,8aS)-6-[rel-(1R,4S,6R)-6-[[2-fluoro-4-(trifluoromethyl)phenyl]methoxy]-2-azabicyclo[2.2.1]heptane-2-carbonyl]-4,4a,5,7,8,8a-hexahydropyrido[4,3-b][1,4]oxazin-3-one;
(4aR,8aS)-6-[rel-(1R,4R,5S)-5-[[2-fluoro-4-(trifluoromethyl)phenyl]methoxy]-2-azabicyclo[2.2.1]heptane-2-carbonyl]-4,4a, 5,7,8,8a-hexahydropyrido[4,3-b][1,4]oxazin-3-one;
(4aR,8aS)-6-[rel-(1R,4R,5S)-5-[[2-fluoro-4-(trifluoromethyl)phenyl]methoxy]-2-azabicyclo[2.2.1]heptane-2-carbonyl]-4,4a,5,7,8,8a-hexahydropyrido[4,3-b][1,4]oxazin-3-one;
(4aR,8aS)-6-[6-[[2-fluoro-4-(trifluoromethyl)phenyl]methoxy]-3-azabicyclo[3.1.1]heptane-3-carbonyl]-4,4a,5,7,8,8a-hexahydropyrido[4,3-b] [1,4]oxazin-3-one;
(4aR,8aS)-6-[(1S,5R)-7-[[2-fluoro-4-(trifluoromethyl)phenyl]methoxy]-3-oxa-9-azabicyclo[3.3.1]nonane-9-carbonyl]-4,4a,5,7,8,8a-hexahydropyrido[4,3-b][1,4]oxazin-3-one;
(4aR,8aS)-6-[(1S,5R)-3-[[2-fluoro-4-(trifluoromethyl)phenyl]methoxy]-8-azabicyclo[3.2.1]octane-8-carbonyl]-4,4a,5,7,8,8a-hexahydropyrido[4,3-b][1,4]oxazin-3-one;
(4aR,8aS)-6-[(1R,5S,6r)-6-[[2-fluoro-4-(trifluoromethyl)phenoxy]methyl]-3-azabicyclo[3.1.0]hexane-3-carbonyl]-4,4a,5,7,8,8a-hexahydropyrido[4,3-b][1,4]oxazin-3-one;
(4aR,8aS)-6-[3-[(2-chloro-4-fluoro-phenoxy)methyl]-8-azabicyclo[3.2.1]octane-8-carbonyl]-4,4a,5,7,8,8a-hexahydropyrido[4,3-b] [1,4]oxazin-3-one;
(4aS,8aR)-6-[8-[(2-chloro-4-fluoro-phenoxy)methyl]-3-azabicyclo[3.2.1]octane-3-carbonyl]-4,4a,5,7,8,8a-hexahydropyrido[4,3-b][1,4]oxazin-3-one; and
(4aR,8aS)-6-[rel-(1R,4R,5S)-5-(5,6,7,8-tetrahydroisoquinolin-3-ylmethoxy)-2-azabicyclo[2.2.1]heptane-2-carbonyl]-4,4a,5,7,8,8a-hexahydropyrido[4,3-b][1,4]oxazin-3-one.

14. A compound of formula (I) according to any one of claims 1 to 12 or a compound of claim 13 for use as therapeutically active substance.

15. A pharmaceutical composition comprising a compound of formula (I) according to any one of claims 1 to 12 or a compound of claim 13 and a therapeutically inert carrier.

16. A compound of formula (I) according to any one of claims 1 to 12 or a compound of claim 13, or a pharmaceutically acceptable salt thereof, or a pharmaceutical composition according to claim 15 for use in the treatment or prophylaxis of neuroinflammation, neurodegenerative diseases, pain, cancer, mental disorders and/or inflammatory bowel disease in a mammal, in particular for use in the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain, spasticity associated with pain, abdominal pain, abdominal pain associated with irritable bowel syndrome and/or visceral pain in a mammal.

## Patentansprüche

1. Verbindung der Formel (I) 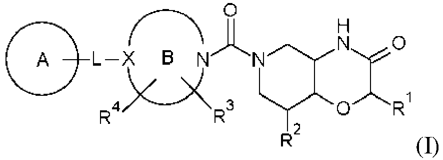 oder ein pharmazeutisch unbedenkliches Salz davon, wobei:
X N oder C-R⁵ ist;
L aus einer kovalenten Bindung, -(CH₂)ₙ-O-, -O-(CH₂)ₚ- und -SO₂- ausgewählt ist;
n eine ganze Zahl, ausgewählt aus 0, 1, 2 und 3, ist;
p eine ganze Zahl, ausgewählt aus 1, 2 und 3, ist;
A (i) mit R⁶, R⁷ und R⁸ substituiertes C₆₋₁₄-Aryl; oder
(ii) mit R⁹, R¹⁰ und R¹¹ substituiertes 5- bis 14-gliedriges Heteroaryl ist; oder
B ein verbrückter bicyclischer Heterocyclus ist;
R¹ Wasserstoff oder C₁₋₆-Alkyl ist;
R² Wasserstoff oder C₁₋₆-Alkyl ist;
R³ Wasserstoff, C₁₋₆-Alkyl, Halogen-C₁₋₆-alkyl, Halogen oder Hydroxy ist;
R⁴ Wasserstoff, C₁₋₆-Alkyl, Halogen-C₁₋₆-alkyl, Halogen oder Hydroxy ist;
R⁵ Wasserstoff, C₁₋₆-Alkyl, Halogen-C₁₋₆-alkyl, Halogen oder Hydroxy ist; und
R⁶, R⁷, R⁸, R⁹, R¹⁰ und R¹¹ unabhängig voneinander aus Wasserstoff, C₁₋₆-Alkyl, Halogen-C₁₋₆-alkyl, Halogen, C₁₋₆-Alkoxy, Halogen-C₁₋₆-alkoxy, SF₅, C₁₋₆-Alkylsulfonyl, Cyano, C₃₋₁₀-Cycloalkyl, C₆₋₁₄-Aryl und 5- bis 14-gliedrigem Heteroaryl ausgewählt sind, wobei das C₃₋₁₀-Cycloalkyl, C₆₋₁₄-Aryl und 5- bis 14-gliedrige Heteroaryl gegebenenfalls mit 1-2 Substituenten substituiert sind, die aus Halogen, Cyano, SF₅ C₁₋₆-Alkyl, C₁₋₆-Alkoxy, Halogen-C₁₋₆-alkyl und Halogen-C₁₋₆-alkoxy ausgewählt sind.

2. Verbindung der Formel (I) nach Anspruch 1 oder ein pharmazeutisch unbedenkliches Salz davon, wobei R¹ und R² beide Wasserstoff sind.

3. Verbindung der Formel (I) nach einem der Ansprüche 1 bis 2 oder ein pharmazeutisch unbedenkliches Salz davon, wobei R³ und R⁴ beide Wasserstoff sind.

4. Verbindung der Formel (I) nach einem der Ansprüche 1 bis 3 oder ein pharmazeutisch unbedenkliches Salz davon, wobei
R⁹ aus Wasserstoff, C₃₋₁₀-Cycloalkyl und C₆₋₁₄-Aryl ausgewählt ist, wobei das C₆₋₁₄-Aryl mit 1-2 Substituenten substituiert ist, die aus Halogen und Halogen-C₁₋₆-alkyl ausgewählt sind;
R¹⁰ aus Wasserstoff und Halogen ausgewählt ist; und
R¹¹ Wasserstoff ist.

5. Verbindung der Formel (I) nach einem der Ansprüche 1 bis 4 oder ein pharmazeutisch unbedenkliches Salz davon, wobei X C-R⁵ ist und R⁵ Wasserstoff ist.

6. Verbindung der Formel (I) nach einem der Ansprüche 1 bis 5 oder ein pharmazeutisch unbedenkliches Salz davon, wobei
L aus -(CH₂)ₙ-O- und -O-(CH₂)ₚ- ausgewählt ist;
n eine ganze Zahl, ausgewählt aus 0 und 1, ist; und
p 1 ist.

7. Verbindung der Formel (I) nach einem der Ansprüche 1 bis 6 oder ein pharmazeutisch unbedenkliches Salz davon, wobei
A mit R⁶, R⁷ und R⁸ substituiertes C₆₋₁₄-Aryl ist;
R⁶ aus Halogen-C₁₋₆-alkyl und Halogen ausgewählt ist;
R⁷ Halogen ist; und
R⁸ Wasserstoff ist.

8. Verbindung der Formel (I) nach Anspruch 7 oder ein pharmazeutisch unbedenkliches Salz davon, wobei
A mit R⁶, R⁷ und R⁸ substituiertes C₆₋₁₄-Aryl ist;
R⁶ aus Wasserstoff, C₁₋₆-Alkyl, Halogen-C₁₋₆-alkyl und Halogen ausgewählt ist;
R⁷ aus Wasserstoff und Halogen ausgewählt ist; und
R⁸ Wasserstoff ist.

9. Verbindung der Formel (I) nach Anspruch 8 oder ein pharmazeutisch unbedenkliches Salz davon, wobei
A mit R⁶, R⁷ und R⁸ substituiertes Phenyl ist;
R⁶ aus CF₃, Chlor und Fluor ausgewählt ist;
R⁷ Fluor oder Chlor ist; und
R⁸ Wasserstoff ist.

10. Verbindung der Formel (I) nach einem der Ansprüche 1 bis 9 oder ein pharmazeutisch unbedenkliches Salz davon, wobei B ein 6- bis 14-gliedriger verbrückter bicyclischer Heterocyclus ist, der 1-3 Heteroatome umfasst, die aus N, S und O ausgewählt sind.

11. Verbindung der Formel (I) nach Anspruch 10 oder ein pharmazeutisch unbedenkliches Salz davon, wobei B ein 6- bis 8-gliedriger verbrückter bicyclischer Heterocyclus ist, der 1 Stickstoffatom umfasst.

12. Verbindung der Formel (I) nach Anspruch 11 oder ein pharmazeutisch unbedenkliches Salz davon, wobei B aus 8-Azabicyclo[3.2.1]octan-8-yl (a); 3,3a,4,5,6,6a-Hexahydro-1H-cyclopenta[c]pyrrol-2-yl (**b**); 3-Azabicyclo[3.1.0]hexan-3-yl (**c**) und 2-Azabicyclo[2.2.1]heptan-2-yl (**d**) ausgewählt ist: 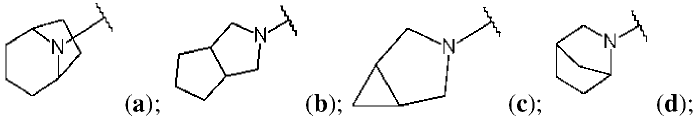 wobei eine Wellenlinie den Bindungspunkt an die Carbonylbrücke, die B mit dem Hexahydropyridooxazinon-Kern der Formel (I) verbrückt, kennzeichnet.

13. Verbindung, ausgewählt aus
(4aR,8aS)-6-(5-(2-Chlorphenoxy)-3,3a,4,5,6,6a-hexahydro-1H-cyclopenta[c]pyrrol-2-carbonyl)-4,4a,5,7,8,8a-hexahydropyrido[4,3-b][1,4]oxazin-3-on;
(4aR,8aS)-6-(5-(2-Methylphenoxy)-3,3a,4,5,6,6a-hexahydro-1H-cyclopenta[c]pyrrol-2-carbonyl)-4,4a,5,7,8,8a-hexahydropyrido[4,3-b][1,4]oxazin-3-on;
(4aR,8aS)-6-(5-(Benzolsulfonyl)-1,3,3a,4,6,6a-hexahydropyrrolo[3,4-c]pyrrol-2-carbonyl)-4,4a,5,7,8,8a-hexahydropyrido[4,3-b][1,4]oxazin-3-on;
(4aR,8aS)-6-(5-[4-(Trifluormethyl)phenyl]sulfonyl-1,3,3a,4,6,6a-hexahydropyrrolo[3,4-c]pyrrol-2-carbonyl)-4,4a,5,7,8,8a-hexahydropyrido[4,3-b][1,4]oxazin-3-on;
(4aR,8aS)-6-(5-(2-Chlor-4-fluorphenyl)sulfonyl-1,3,3a,4,6,6a-hexahydropyrrolo[3,4-c]pyrrol-2-carbonyl)-4,4a,5,7,8,8a-hexahydropyrido[4,3-b][1,4]oxazin-3-on;
(4aR,8aS)-6-[(1R,5S,6r)-6-[1-(4-Fluorphenyl)pyrazol-3-yl]-3-azabicyclo[3.1.0]hexan-3-carbonyl]-4,4a,5,7,8,8a-hexahydropyrido[4,3-b] [1,4]oxazin-3-on;
(4aR,8aS)-6-[(1R,5S,6r)-6-[1-[4-Fluor-3-(trifluormethyl)phenyl]pyrazol-3-yl]-3-azabicyclo[3.1.0]hexan-3-carbonyl]-4,4a,5,7,8,8a-hexahydropyrido[4,3-b][1,4]oxazin-3-on;
(4aR,8aS)-6-[(1R,5S,6r)-6-[1-(2-Chlor-4-fluorphenyl)pyrazol-3-yl]-3-azabicyclo[3.1.0]hexan-3-carbonyl]-4,4a,5,7,8,8a-hexahydropyrido[4,3-b][1,4]oxazin-3-on;
(4aR,8aS)-6-[rel-(1R,4R,5S)-5-(5,6,7,8-Tetrahydroisochinolin-3-ylmethoxy)-2-azabicyclo[2.2.1]heptan-2-carbonyl]-4,4a,5,7,8,8a-hexahydropyrido[4,3-b][1,4]oxazin-3-on;
(4aR,8aS)-6-[rel-(1R,4R,5S)-5-[(5-Chlor-4-cyclopropyl-2-pyridyl)methoxy]-2-azabicyclo[2.2.1]heptan-2-carbonyl]-4,4a,5,7,8,8a-hexahydropyrido[4,3-b][1,4]oxazin-3-on;
(4aR,8aS)-6-[rel-(1R,4R,5S)-5-[(5-Chlor-4-cyclopropyl-2-pyridyl)methoxy]-2-azabicyclo[2.2.1]heptan-2-carbonyl]-4,4a,5,7,8,8a-hexahydropyrido[4,3-b][1,4]oxazin-3-on;
(4aR,8aS)-6-[(1R,5S,6r)-6-[(2-Chlor-4-fluorphenoxy)methyl]-3-azabicyclo[3.1.0]hexan-3-carbonyl]-4,4a,5,7,8,8a-hexahydropyrido[4,3-b][1,4]oxazin-3-on;
(4aR,8aS)-6-[8-[(2-Chlor-4-fluorphenoxy)methyl]-3-azabicyclo[3.2.1]octan-3-carbonyl]-4,4a,5,7,8,8a-hexahydropyrido[4,3-b] [1,4]oxazin-3-on;
(4aR,8aS)-6-[5-(2-Chlor-4-fluorphenoxy)-3,3a,4,5,6,6a-hexahydro-1H-cyclopenta[c]pyrrol-2-carbonyl]-4,4a,5,7,8,8a-hexahydropyrido[4,3-b][1,4]oxazin-3-on;
(4aR,8aS)-6-[5-[(2-Chlor-4-fluorphenyl)methoxy]-3,3a,4,5,6,6a-hexahydro-1H-cyclopenta[c]pyrrol-2-carbonyl]-4,4a,5,7,8,8a-hexahydropyrido[4,3-b][1,4]oxazin-3-on;
(4aR,8aS)-6-[5-[[2-Fluor-4-(trifluormethyl)phenyl]methoxy]-3,3a,4,5,6,6a-hexahydro-1H-cyclopenta[c]pyrrol-2-carbonyl]-4,4a,5,7,8,8a-hexahydropyrido[4,3-b][1,4]oxazin-3-on;
(4aR,8aS)-6-[5-[2-Fluor-4-(trifluormethyl)phenoxy]-3,3a,4,5,6,6a-hexahydro-1H-cyclopenta[c]pyrrol-2-carbonyl]-4,4a,5,7,8,8a-hexahydropyrido[4,3-b][1,4]oxazin-3-on;
(4aR,8aS)-6-[rel-(1R,4S,6S)-6-[[2-Fluor-4-(trifluormethyl)phenyl]methoxy]-2-azabicyclo[2.2.1]heptan-2-carbonyl]-4,4a,5,7,8,8a-hexahydropyrido[4,3-b][1,4]oxazin-3-on;
(4aR,8aS)-6-[rel-(1R,4S,6R)-6-[[2-Fluor-4-(trifluormethyl)phenyl]methoxy]-2-azabicyclo[2.2.1]heptan-2-carbonyl]-4,4a,5,7,8,8a-hexahydropyrido[4,3-b][1,4]oxazin-3-on;
(4aR,8aS)-6-[rel-(1R,4R,SS)-5-[[2-Fluor-4-(trifluormethyl)phenyl]methoxy]-2-azabicyclo[2.2.1]heptan-2-carbonyl]-4,4a,5,7,8,8a-hexahydropyrido[4,3-b][1,4]oxazin-3-on;
(4aR,8aS)-6-[rel-(1R,4R,SS)-5-[[2-Fluor-4-(trifluormethyl)phenyl]methoxy]-2-azabicyclo[2.2.1]heptan-2-carbonyl]-4,4a,5,7,8,8a-hexahydropyrido[4,3-b][1,4]oxazin-3-on;
(4aR,8aS)-6-[6-[[2-Fluor-4-(trifluormethyl)phenyl]methoxy]-3-azabicyclo[3.1.1]heptan-3-carbonyl]-4,4a,5,7,8,8a-hexahydropyrido[4,3-b] [1,4]oxazin-3-on;
(4aR,8aS)-6-[(1S,5R)-7-[[2-Fluor-4-(trifluormethyl)phenyl]methoxy]-3-oxa-9-azabicyclo[3.3.1]nonan-9-carbonyl]-4,4a,5,7,8,8a-hexahydropyrido[4,3-b][1,4]oxazin-3-on;
(4aR,8aS)-6-[(1S,5R)-3-[[2-Fluor-4-(trifluormethyl)phenyl]methoxy]-8-azabicyclo[3.2.1]octan-8-carbonyl]-4,4a,5,7,8,8a-hexahydropyrido[4,3-b][1,4]oxazin-3-on;
(4aR,8aS)-6-[(1R,5S,6r)-6-[[2-Fluor-4-(trifluormethyl)phenoxy]methyl]-3-azabicyclo[3.1.0]hexan-3-carbonyl]-4,4a,5,7,8,8a-hexahydropyrido[4,3-b][1,4]oxazin-3-on;
(4aR,8aS)-6-[3-[(2-Chlor-4-fluorphenoxy)methyl]-8-azabicyclo[3.2.1]octan-8-carbonyl]-4,4a,5,7,8,8a-hexahydropyrido[4,3-b][1,4]oxazin-3-on;
(4aS,8aR)-6-[8-[(2-Chlor-4-fluorphenoxy)methyl]-3-azabicyclo[3.2.1]octan-3-carbonyl]-4,4a,5,7,8,8a-hexahydropyrido[4,3-b][1,4]oxazin-3-on; und
(4aR,8aS)-6-[rel-(1R,4R,5S)-5-(5,6,7,8-Tetrahydroisochinolin-3-ylmethoxy)-2-azabicyclo[2.2.1]heptan-2-carbonyl]-4,4a,5,7,8,8a-hexahydropyrido[4,3-b][1,4]oxazin-3-on.

14. Verbindung der Formel (I) nach einem der Ansprüche 1 bis 12 oder Verbindung nach Anspruch 13 zur Verwendung als therapeutisch wirksame Substanz.

15. Pharmazeutische Zusammensetzung, umfassend eine Verbindung der Formel (I) nach einem der Ansprüche 1 bis 12 oder eine Verbindung nach Anspruch 13 und einen therapeutisch inerten Träger.

16. Verbindung der Formel (I) nach einem der Ansprüche 1 bis 12 oder Verbindung nach Anspruch 13 oder ein pharmazeutisch unbedenkliches Salz davon oder pharmazeutische Zusammensetzung nach Anspruch 15 zur Verwendung bei der Behandlung oder Prophylaxe von Neuroinflammation, neurodegenerativen Krankheiten, Schmerz, Krebs, mentalen Störungen und/oder entzündlicher Darmkrankheit bei einem Säuger, insbesondere zur Verwendung bei der Behandlung oder Prophylaxe von multipler Sklerose, Alzheimer-Krankheit, Parkinson-Krankheit, amyotropher Lateralsklerose, traumatischer Hirnverletzung, Neurotoxizität, Schlaganfall, Epilepsie, Angst, Migräne, Depression, Leberzellkrebs, Kolonkarzinogenese, Eierstockkrebs, neuropathischem Schmerz, durch Chemotherapie induzierter Neuropathie, akutem Schmerz, chronischem Schmerz, Spastizität in Verbindung mit Schmerz, Bauchschmerz, Bauchschmerz in Verbindung mit Reizdarmsyndrom und/oder viszeralem Schmerz bei einem Säuger.

## Revendications

1. Composé de formule (I) 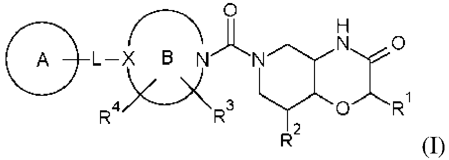 ou un sel pharmaceutiquement acceptable de celui-ci, dans lequel :
X représente N ou C-R⁵ ;
L est choisi parmi une liaison covalente, -(CH₂)ₙ-O-, -O-(CH₂)ₚ- et -SO₂- ;
n représente un entier choisi parmi 0, 1, 2 et 3 ;
p représente un entier choisi parmi 1, 2 et 3 ;
A représente :
(i) un aryle en C₆₋₁₄ substitué par R⁶, R⁷ et R⁸ ; ou
(ii) un hétéroaryle à 5 à 14 chaînons substitué par R⁹, R¹⁰ et R¹¹ ; ou
B représente un hétérocycle bicyclique ponté ;
R¹ représente un hydrogène ou un alkyle en C₁₋₆ ;
R² représente un hydrogène ou un alkyle en C₁₋₆ ;
R³ représente un hydrogène, un alkyle en C₁₋₆, un halogénoalkyle en C₁₋₆, un halogène ou un hydroxy ;
R⁴ représente un hydrogène, un alkyle en C₁₋₆, un halogénoalkyle en C₁₋₆, un halogène ou un hydroxy ;
R⁵ représente un hydrogène, un alkyle en C₁₋₆, un halogénoalkyle en C₁₋₆, un halogène ou un hydroxy ; et
R⁶, R⁷, R⁸, R⁹, R¹⁰ et R¹¹ sont indépendamment choisis parmi un hydrogène, un alkyle en C₁₋₆, un halogénoalkyle en C₁₋₆, un halogène, un alcoxy en C₁₋₆, un halogénoalcoxy en C₁₋₆, SF₅, un alkyle en C₁₋₆-sulfonyle, un cyano, un cycloalkyle en C₃₋₁₀, un aryle en C₆₋₁₄ et un hétéroaryle à 5 à 14 chaînons, dans lequel lesdits cycloalkyle en C₃₋₁₀, aryle en C₆₋₁₄ et hétéroaryle à 5 à 14 chaînons sont éventuellement substitués par 1 à 2 substituants choisis parmi un halogène, un cyano, SF₅ un alkyle en C₁₋₆, un alcoxy en C₁₋₆, un halogénoalkyle en C₁₋₆ et un halogénoalcoxy en C₁₋₆.

2. Composé de formule (I) selon la revendication 1, ou un sel pharmaceutiquement acceptable de celui-ci, dans lequel R¹ et R² représentent tous deux un hydrogène.

3. Composé de formule (I) selon l'une quelconque des revendications 1 à 2, ou un sel pharmaceutiquement acceptable de celui-ci, dans lequel R³ et R⁴ représentent tous deux un hydrogène.

4. Composé de formule (I) selon l'une quelconque des revendications 1 à 3, ou un sel pharmaceutiquement acceptable de celui-ci, dans lequel
R⁹ est choisi parmi un hydrogène, un cycloalkyle en C₃₋₁₀ et un aryle en C₆₋₁₄, dans lequel ledit aryle en C₆₋₁₄ est substitué par 1 à 2 substituants choisis parmi un halogène et un halogénoalkyle en C₁₋₆ ;
R¹⁰ est choisi parmi un hydrogène et un halogène ; et
R¹¹ représente un hydrogène.

5. Composé de formule (I) selon l'une quelconque des revendications 1 à 4, ou un sel pharmaceutiquement acceptable de celui-ci, dans lequel X représente C-R⁵ et R⁵ représente un hydrogène.

6. Composé de formule (I) selon l'une quelconque des revendications 1 à 5, ou un sel pharmaceutiquement acceptable de celui-ci, dans lequel
L est choisi parmi -(CH₂)ₙ-O- et -O-(CH₂)ₚ- ;
n représente un entier choisi parmi 0 et 1 ; et
p représente 1.

7. Composé de formule (I) selon l'une quelconque des revendications 1 à 6, ou un sel pharmaceutiquement acceptable de celui-ci, dans lequel
A représente un aryle en C₆₋₁₄ substitué par R⁶, R⁷ et R⁸ ;
R⁶ est choisi parmi un halogénoalkyle en C₁₋₆ et un halogène ;
R⁷ représente un halogène ; et
R⁸ représente un hydrogène.

8. Composé de formule (I) selon la revendication 7, ou un sel pharmaceutiquement acceptable de celui-ci, dans lequel
A représente un aryle en C₆₋₁₄ substitué par R⁶, R⁷ et R⁸ ;
R⁶ est choisi parmi un hydrogène, un alkyle en C₁₋₆, un halogénoalkyle en C₁₋₆ et un halogène ;
R⁷ est choisi parmi un hydrogène et un halogène ; et
R⁸ représente un hydrogène.

9. Composé de formule (I) selon la revendication 8, ou un sel pharmaceutiquement acceptable de celui-ci, dans lequel
A représente un phényle substitué par R⁶, R⁷ et R⁸ ;
R⁶ est choisi parmi CF₃, un chlore et un fluor ;
R⁷ représente un fluor ou un chlore ; et
R⁸ représente un hydrogène.

10. Composé de formule (I) selon l'une quelconque des revendications 1 à 9, ou un sel pharmaceutiquement acceptable de celui-ci, dans lequel B représente un hétérocycle bicyclique ponté à 6 à 14 chaînons comprenant 1 à 3 hétéroatomes choisis parmi N, S et O.

11. Composé de formule (I) selon la revendication 10, ou un sel pharmaceutiquement acceptable de celui-ci, dans lequel B représente un hétérocycle bicyclique ponté à 6 à 8 chaînons comprenant 1 atome d'azote.

12. Composé de formule (I) selon la revendication 11, ou un sel pharmaceutiquement acceptable de celui-ci, dans lequel B est choisi parmi un 8-azabicyclo[3.2.1]octan-8-yle (a) ; un 3,3a,4,5,6,6a-hexahydro-1H-cyclopenta[c]pyrrol-2-yle (b) ; un 3-azabicyclo[3.1.0]hexan-3-yle (c) ; et un 2-azabicyclo[2.2.1]heptan-2-yle (d) : 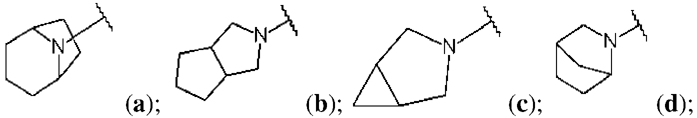 dans lequel un trait ondulé indique le point de liaison au pont carbonyle reliant B au noyau hexahydropyrido oxazinone de formule (I).

13. Composé choisi parmi
la (4aR,8aS)-6-(5-(2-chlorophénoxy)-3,3a,4,5,6,6a-hexahydro-1H-cyclopenta[c]pyrrole-2-carbonyl)-4,4a,5,7,8,8a-hexahydropyrido[4,3-b][1,4]oxazin-3-one ;
la (4aR,8aS)-6-(5-(2-méthylphénoxy)-3,3a,4,5,6,6a-hexahydro-1H-cyclopenta[c]pyrrole-2-carbonyl)-4,4a,5,7,8,8a-hexahydropyrido[4,3-b] [1,4]oxazin-3-one ;
la (4aR,8aS)-6-(5-(benzènesulfonyl)-1,3,3a,4,6,6a-hexahydropyrrolo[3,4-c]pyrrole-2-carbonyl)-4,4a,5,7,8,8a-hexahydropyrido[4,3-b][1,4]oxazin-3-one ;
la (4aR,8aS)-6-(5-[4-(trifluorométhyl)phényl]sulfonyl-1,3,3a,4,6,6a-hexahydropyrrolo[3,4-c]pyrrole-2-carbonyl)-4,4a,5,7,8,8a-hexahydropyrido[4,3-b][1,4]oxazin-3-one ;
la (4aR,8aS)-6-(5-(2-chloro-4-fluoro-phényl)sulfonyl-1,3,3a,4,6,6a-hexahydropyrrolo[3,4-c]pyrrole-2-carbonyl)-4,4a,5,7,8,8a-hexahydropyrido[4,3-b][1,4]oxazin-3-one ;
la (4aR,8aS)-6-[(1R,5S,6r)-6-[1-(4-fluorophényl)pyrazol-3-yl]-3-azabicyclo[3.1.0]hexane-3-carbonyl]-4,4a,5,7,8,8a-hexahydropyrido[4,3-b][1,4]oxazin-3-one ;
la (4aR,8aS)-6-[(1R,SS,6r)-6-[1-[4-fluoro-3-(trifluorométhyl)phényl]pyrazol-3-yl]-3-azabicyclo[3.1.0]hexane-3-carbonyl]-4,4a,5,7,8,8a-hexahydropyrido[4,3-b][1,4]oxazin-3-one ;
la (4aR,8aS)-6-[(1R,5S,6r)-6-[1-(2-chloro-4-fluoro-phényl)pyrazol-3-yl]-3-azabicyclo[3.1.0]hexane-3-carbonyl]-4,4a,5,7,8,8a-hexahydropyrido[4,3-b][1,4]oxazin-3-one ;
la (4aR,8aS)-6-[rel-(1R,4R,5S)-5-(5,6,7,8-tétrahydroisoquinoléin-3-ylméthoxy)-2-azabicyclo[2.2.1]heptane-2-carbonyl]-4,4a,5,7,8,8a-hexahydropyrido[4,3-b][1,4]oxazin-3-one ;
la (4aR,8aS)-6-[rel-(1R,4R,5S)-5-[(5-chloro-4-cyclopropyl-2-pyridyl)méthoxy]-2-azabicyclo[2.2.1]heptane-2-carbonyl]-4,4a,5,7,8,8a-hexahydropyrido[4,3-b][1,4]oxazin-3-one ;
la (4aR,8aS)-6-[rel-(1R,4R,5S)-5-[(5-chloro-4-cyclopropyl-2-pyridyl)méthoxy]-2-azabicyclo[2.2.1]heptane-2-carbonyl]-4,4a,5,7,8,8a-hexahydropyrido[4,3-b][1,4]oxazin-3-one ;
la (4aR,8aS)-6-[(1R,5S,6r)-6-[(2-chloro-4-fluoro-phénoxy)méthyl]-3-azabicyclo[3.1.0]hexane-3-carbonyl]-4,4a,5,7,8,8a-hexahydropyrido[4,3-b][1,4]oxazin-3-one ;
la (4aR,8aS)-6-[8-[(2-chloro-4-fluoro-phénoxy)méthyl]-3-azabicyclo[3.2.1]octane-3-carbonyl]-4,4a,5,7,8,8a-hexahydropyrido[4,3-b][1,4]oxazin-3-one ;
la (4aR,8aS)-6-[5-(2-chloro-4-fluoro-phénoxy)-3,3a,4,5,6,6a-hexahydro-1H-cyclopenta[c]pyrrole-2-carbonyl]-4,4a,5,7,8,8a-hexahydropyrido[4,3-b] [1,4]oxazin-3-one ;
la (4aR,8aS)-6-[5-[(2-chloro-4-fluoro-phényl)méthoxy]-3,3a,4,5,6,6a-hexahydro-1H-cyclopenta[c]pyrrole-2-carbonyl]-4,4a,5,7,8,8a-hexahydropyrido[4,3-b] [1,4]oxazin-3-one ;
la (4aR,8aS)-6-[5-[[2-fluoro-4-(trifluorométhyl)phényl]méthoxy]-3,3a,4,5,6,6a-hexahydro-1H-cyclopenta[c]pyrrole-2-carbonyl]-4,4a,5,7,8,8a-hexahydropyrido[4,3-b][1,4]oxazin-3-one ;
la (4aR,8aS)-6-[5-[2-fluoro-4-(trifluorométhyl)phénoxy]-3,3a,4,5,6,6a-hexahydro-1H-cyclopenta[c]pyrrole-2-carbonyl]-4,4a,5,7,8,8a-hexahydropyrido[4,3-b] [1,4]oxazin-3-one ;
la (4aR,8aS)-6-[rel-(1R,4S,6S)-6-[[2-fluoro-4-(trifluorométhyl)phényl]méthoxy]-2-azabicyclo[2.2.1]heptane-2-carbonyl]-4,4a,5,7,8,8a-hexahydropyrido[4,3-b][1,4]oxazin-3-one ;
la (4aR,8aS)-6-[rel-(1R,4S,6R)-6-[[2-fluoro-4-(trifluorométhyl)phényl]méthoxy]-2-azabicyclo[2.2.1]heptane-2-carbonyl]-4,4a,5,7,8,8a-hexahydropyrido[4,3-b][1,4]oxazin-3-one ;
la (4aR,8aS)-6-[rel-(1R,4R,5S)-5-[[2-fluoro-4-(trifluorométhyl)phényl]méthoxy]-2-azabicyclo[2.2.1]heptane-2-carbonyl]-4,4a,5,7,8,8a-hexahydropyrido[4,3-b][1,4]oxazin-3-one ;
la (4aR,8aS)-6-[rel-(1R,4R,5S)-5-[[2-fluoro-4-(trifluorométhyl)phényl]méthoxy]-2-azabicyclo[2.2.1]heptane-2-carbonyl]-4,4a,5,7,8,8a-hexahydropyrido[4,3-b][1,4]oxazin-3-one ;
la (4aR,8aS)-6-[6-[[2-fluoro-4-(trifluorométhyl)phényl]méthoxy]-3-azabicyclo[3.1.1]heptane-3-carbonyl]-4,4a,5,7,8,8a-hexahydropyrido[4,3-b] [1,4]oxazin-3-one ;
la (4aR,8aS)-6-[(1S,5R)-7-[[2-fluoro-4-(trifluorométhyl)phényl]méthoxy]-3-oxa-9-azabicyclo[3.3.1]nonane-9-carbonyl]-4,4a,5,7,8,8a-hexahydropyrido[4,3-b][1,4]oxazin-3-one ;
la (4aR,8aS)-6-[(1S,5R)-3-[[2-fluoro-4-(trifluorométhyl)phényl]méthoxy]-8-azabicyclo[3.2.1]octane-8-carbonyl]-4,4a,5,7,8,8a-hexahydropyrido[4,3-b][1,4]oxazin-3-one ;
la (4aR,8aS)-6-[(1R,5S,6r)-6-[[2-fluoro-4-(trifluorométhyl)phénoxy]méthyl]-3-azabicyclo[3.1.0]hexane-3-carbonyl]-4,4a,5,7,8,8a-hexahydropyrido[4,3-b][1,4]oxazin-3-one ;
la (4aR,8aS)-6-[3-[(2-chloro-4-fluoro-phénoxy)méthyl]-8-azabicyclo[3.2.1]octane-8-carbonyl]-4,4a,5,7,8,8a-hexahydropyrido[4,3-b][1,4]oxazin-3-one ;
la (4aS,8aR)-6-[8-[(2-chloro-4-fluoro-phénoxy)méthyl]-3-azabicyclo[3.2.1]octane-3-carbonyl]-4,4a,5,7,8,8a-hexahydropyrido[4,3-b][1,4]oxazin-3-one ; et
la (4aR,8aS)-6-[rel-(1R,4R,5S)-5-(5,6,7,8-tétrahydroisoquinoléin-3-ylméthoxy)-2-azabicyclo[2.2.1]heptane-2-carbonyl]-4,4a,5,7,8,8a-hexahydropyrido[4,3-b][1,4]oxazin-3-one.

14. Composé de formule (I) selon l'une quelconque des revendications 1 à 12 ou composé selon la revendication 13 pour une utilisation comme substance thérapeutiquement active.

15. Composition pharmaceutique comprenant un composé de formule (I) selon l'une quelconque des revendications 1 à 12 ou un composé selon la revendication 13 et un véhicule thérapeutiquement inerte.

16. Composé de formule (I) selon l'une quelconque des revendications 1 à 12 ou composé selon la revendication 13, ou un sel pharmaceutiquement acceptable de celui-ci, ou composition pharmaceutique selon la revendication 15 pour une utilisation dans le traitement ou la prophylaxie d'une neuro-inflammation, de maladies neurodégénératives, d'une douleur, d'un cancer, de troubles mentaux et/ou d'une maladie inflammatoire de l'intestin chez un mammifère, en particulier pour une utilisation dans le traitement ou la prophylaxie d'une sclérose en plaques, de la maladie d'Alzheimer, de la maladie de Parkinson, d'une sclérose latérale amyotrophique, d'une lésion cérébrale traumatique, d'une neurotoxicité, d'un accident vasculaire cérébral, d'une épilepsie, d'une anxiété, d'une migraine, d'une dépression, d'un carcinome hépatocellulaire, d'une carcinogenèse du côlon, d'un cancer de l'ovaire, d'une douleur neuropathique, d'une neuropathie induite par la chimiothérapie, d'une douleur aiguë, d'une douleur chronique, d'une spasticité associée à la douleur, d'une douleur abdominale, d'une douleur abdominale associée au syndrome de l'intestin irritable et/ou d'une douleur viscérale chez un mammifère.
